(19) Europäisches Patentamt
European Patent Office
Office européen des brevets

(11) EP 0 714 662 A1

(12) **EUROPEAN PATENT APPLICATION**
published in accordance with Art. 158(3) EPC

(43) Date of publication:
**05.06.1996 Bulletin 1996/23**

(21) Application number: **94921100.7**

(22) Date of filing: **15.07.1994**

(51) Int. Cl.[6]: **A61K 37/02**, A61K 39/35, A61K 39/36

(86) International application number:
**PCT/JP94/01164**

(87) International publication number:
**WO 95/02412 (26.01.1995 Gazette 1995/05)**

(84) Designated Contracting States:
**AT BE CH DE DK ES FR GB GR IE IT LI LU MC NL PT SE**

(30) Priority: **16.07.1993 JP 177008/93**
**01.09.1993 JP 217725/93**
**07.04.1994 JP 69336/94**

(71) Applicant: **MEIJI MILK PRODUCTS COMPANY LIMITED**
**Tokyo 104 (JP)**

(72) Inventors:
- **KINO, Kohsuke**
**Odawara-shi Kanagawa 250 (JP)**
- **KOMIYAMA, Naoki**
**Odawara-shi Kanagawa 250 (JP)**
- **SONE, Toshio**
**Odawara-shi Kanagawa 250 (JP)**
- **KOHNO, Yoichi**
**Chiba-shi Chiba 261 (JP)**

(74) Representative: **Wächtershäuser, Günter, Prof. Dr.**
**Patentanwalt,**
**Tal 29**
**D-80331 München (DE)**

# (54) ANTIALLERGIC AGENT

(57) An antiallergic agent containing as the active ingredient a peptide containing a monovalent B cell epitope on an allergen molecule with which an IgE antibody specifically combines and having the effect of inhibiting the formation of a cross-linked structure by the combination of the allergen with the IgE antibody. This agent works on the basis of an entirely new mechanism of inhibiting antigen-specifically the cross-linking of the IgE antibody. It is useful for preventing and treating immediate allergic diseases such as food allergy, tick allergy, bronchial asthma, pollinosis, allergic rhinitis and allergic enterogastritis.

Fig. 3

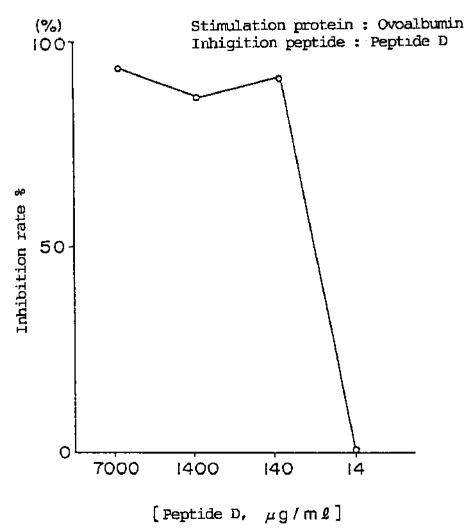


EP 0 714 662 A1

## Description

Technical Field:

The present invention relates to anti-allergic agents effective for preventing and treating type I allergic diseases.

Technical Background:

According to a survey conducted by the Japan Ministry of Health and Welfare in May 1992, the number of patients suffering from allergic diseases is increasing. It is said that one third of the population in Japan displays some allergy symptoms including minor allergies.

It is also said that the increase in the population suffering from allergic diseases relates, either directly or indirectly, to an increase of mites and allergens in house dust resulting from changes in the residential and living environment, changes in diet, stress, and air pollution.

Although allergic diseases have a wide variety of symptoms, most of them are categorized as type I allergies (also called immediate allergies), and type II through IV allergies are rather rare. Type I allergies include bronchial asthma, atopic dermatitis, pollinosis, allergic enterogastritis, food allergies, and anaphylactic shock.

Antigens that cause an allergic reaction are called allergens. Major allergens (which may be referred to in this description as antigens) include foods such as milk, eggs, and soybeans; pollens of cryptomeria, Japanese cypress, white birch, and hogweed; and mites in house dust that inhabit the living environment. These allergens frequently induce onset of allergic diseases.

Type I allergies are prevented and treated by the administration of anti-allergic drugs, hyposensitization therapy using the action of the immune system, and diet therapy or low-allergen food treatment in food allergies.

(1) Anti-allergic drugs:

The onset mechanism of type I allergies has the following three stages:

(a) Antigens which have entered the human body are encapsulated by antigen-presenting cells, which lyse the antigens into peptides. The peptides form complexes with their own MHC proteins, thereby transmitting their antigenity to activate T cells. The activated T cells accelerate differentiation and progression of B cells, and induce production of IgE antibodies which are antigen-specific. The IgE antibodies thus produced are bound to IgE receptors on basophils and mast cells in tissue.

(b) Subsequently, when the same antigen invade into the body again, 2 molecules of IgE antibodies on mast cells react with the antigen, allowing IgE receptors to crosslink via IgE antibodies. This cross-linking phenomenon triggers flowing of $Ca^{++}$ into cells as well as activation of cell membrane enzymes, releasing chemical transmitters including histamine, SRS-A (mixtures of leukotrienes C4, D4, and E4), prostaglandin, thromboxane, and PAF.

(c) Histamine, SRS-A, etc. cause an increase in vascular permeability, mucosal hyper secretion, and contraction of smooth muscle, producing allergic symptoms.

Most anti-allergy drugs currently used in clinical situations for the onset mechanism of type I allergies act during the second stage, and are divided into two groups: acidic anti-allergic drugs which suppress release of chemical transmitters from mast cells, and basic anti-allergic drugs having, in addition to this effect, antihistaminic action. Drugs acting in the third stage include antagonists against chemical transmitters and smooth muscle relaxants. Presently, there are scarecely drugs which suppress IgE antibody production in the first stage and which are used in clinical fields. Only one such drug, IPD-1151T (by Taiho Pharmaceutical Co.), is in the development stage.

(2) Hyposensitization therapy

In hyposensitization therapy, sensitivity toward pathogenic allergens is reduced by subcutaneously injecting causal allergens of an immediate allergy in small amounts a plurality of times.

Hyposensitization therapy is an old therapy, and is used as therapy for atopic bronchial asthma and allergic rhinitis. Although this is an excellent therapy, it has the following drawbacks: patients tend to fear injection, frequent visits and long-term treatment are needed, and the risk of systemic side effects has not yet been eliminated. In order to solve these problems, hyposensitization by the oral route is now being attempted. However, its efficacy is doubtful.

(3) Diet therapy

Allergic symptoms can be mitigated by a diet therapy which eliminates causal food allergens from the daily diet. However, allergens are not easily identified, and in addition, in many cases a plurality of food allergens co-exist, which may concurrently induce allergic reactions. In such a case, malnutrition may result.

(4) Low allergen food

Low allergen foods have been developed in which T-cell epitopes and B-cell epitopes in food allergens are identified, and such epitope regions are eliminated using protease or a like substance, by cleaving, or by modifying, in order to reduce the activity of allergens. For example, low allergen foods prepared from milk are already on the market. Also, development is on the way of low allergen foods prepared from rice, eggs, and soya beans.

However, low allergen foods have shortcomings in taste (bitter taste) and odor. Moreover, they do not necessarily work on any patients.

On the other hand, anti-allergic agents used in clinical situations are all based on a symptomatic approach, and their goal is to mitigate allergic symptoms. They are found less effective than expected, IPD-1151T, which is currently under development, also is based on a mechanism of suppressing the production of IgE antibodies in an antigen-non-specific manner.

Accordingly, an object of the present invention is to provide an anti-allergic drug which inhibits release of chemical transmitters in an antigen-specific manner, thus not altering the normal immune response against other exogenous antigens.

Disclosure of the Invention:

The present inventors found that, since the onset of type I allergic diseases is caused by the release of chemical transmitters such as histamine locally or systemically acting to cause allergic symptoms triggered by the formation of cross-linking when two IgE antibody molecules bound to the surfaces of mast cells or basophils are reacted with one molecule of re-invading allergen, a substance that inhibits the bonding of an IgE antibody with an allergen can be an anti-allergic agent having a novel working mechanism. The present invention was completed based on this idea.

On an allergen molecule there are present a plurality of independent B cell epitopes (polyvalent epitopes) to which IgE antibodies are specifically bound. It is considered that a peptide fragment molecule containing a monovalent B cell epitope is bound only to its counterpart IgE antibody molecule on mast cells and basophils sensitized by the allergen. Therefore, if such a peptide fragment is synthesized and administered to a subject, binding of an allergen and its counterpart IgE antibody can be inhibited in an antigen-specific manner to suppress formation of a cross-linking structure which is indispensable for the onset of I type allergic reactions. In this case, since the epitopes on an allergen molecule which are bound to two IgE antibody molecules are different from each other, peptide fragments each containing only one of the two epitopes can also inhibit formation of a cross-linking structure.

Accordingly, the present invention relates to an anti-allergic agent comprising, as an active component, a peptide containing a monovalent B cell epitope present on an allergen, the epitope being specifically bound to an IgE antibody, and having an effect of inhibiting formation of a cross-linking structure with the allergen and the IgE antibody.

The present invention also relates to a medicinal composition for preventing and treating allergic diseases which comprised an effective amount of the peptide and a pharmaceutically acceptable carrier.

The present invention further relates to a method for preventing and treating allergic diseases which is characterized by administering an effective amount of the peptide.

The present invention further relates to use of the above peptide as a medicine (particularly as a medicine for preventing and treating allergic diseases).

Brief Description of Drawings:

Fig. 1 shows an amino acid sequence of OVA. Fig. 2 is a graph showing the inhibition with peptide D against histamine release caused by stimulation by peptide B. Fig. 3 is a graph showing the inhibition with peptide D against histamine release caused by stimulation by OVA. Fig. 4 is a graph showing the frequency of binding reactions of 61 kinds of overlapping peptides of a Der p II allergen and serum IgE from 15 patients suffering from mite rhinallergosis. Fig. 5 is a graph showing the inhibition of binding of IgE in pooled serum from patients suffering from mite rhinallergosis and mite body extract by the addition of Der p II peptide (55-63). Fig. 6 is a graph showing inhibition of a Der p II peptide (55-63) in a histamine release reaction caused by an antigen (MBE). Fig. 7 shows: (A) an amino acid sequence of 10 residues from the N-terminal of a cryptomeria pollen allergen Cry j II; (B) a DNA sequence deduced from 10 residues from the N-terminal of a cryptomeria (Japanese cedar) pollen allergen Cry j II; and (C) the structure of a probe for screening cDNA coding for a cryptomeria pollen allergen Cry j II. Fig. 8 shows Western blotting profiles of the antigenity of T7 Cry j II identified using each serum of two patients suffering cryptomeria pollinosis. Lane 1, lane 2, lane 3, and lane 4 indicate, respectively, BL21(DE3) which holds pMGEMEX-1 (negative control), BL21(DE3) which expresses T7 Cry j I, BL21(DE3) which expresses T7 Cry j II, and Cry j I purified from cryptomeria pollens.

Best Mode for Carrying Out the Invention:

The peptide used in the present invention (1) contains a monovalent B cell epitope which is found in an allergen molecule, the epitope being specifically bound to an IgE antibody, and (2) has the action of inhibiting the formation of cross-linking caused by binding of the allergen and the IgE antibody.

In order to synthesize a peptide containing a monovalent B cell epitope, B cell epitopes in an allergen molecule must be first identified. This can be done, for example, using a method described below in which the possible locations of B cell epitopes in an allergen molecule are narrowed down to identify their exact locations, after the allergen is fragmentated with a protease and then allowed to react with a serum IgE antibody. However, if the entire primary structure of an allergen is known, a peptide containing monovalent B cell epitopes may be identified by the following method

Peptide fragments each consisting of 4 to 10 amino acids covering the entire primary structure of an allergen are chemically synthesized using a peptide synthesizer. At this time, it is necessary that each overlapping portion of peptides have the size of 3 to 8 residues so as to prevent B cell epitope portions from being divided.

In order to screen such overlapping peptides so as to obtain peptides containing only target monovalent B cell epitopes, the following method based on an antigen-antibody reaction may be used.

(1) Primary screening by an enzyme antibody method:

To perform primary screening of B cell epitopes using a reaction of a peptide and a serum IgE antibody, the following enzyme antibody method is used, by which many samples can be quickly screened. Serum IgE antibodies from a patient having a RAST value of 4 or more react with a peptide in a 96-well plate at room temperature, after which the reaction is washed with a buffer (pH 7.5) to remove the patient's serum. Subsequently, enzyme-labelled anti-human IgE antibodies are added over night at room temperature, followed by washing with the same buffer as above. A 4-methylumberypheryl-β-D-galactopyranoside Solution is added to the 96-well plate to develop a color. Absorbance is measured in each well using a fluorospectrophotometer. Some peptides react with patient's serum IgE antibodies attributed to allergens from which the peptides have been derived These peptides are considered to take a primary structure in view of the reaction conditions in the synthesizing process. Thus, patient's serum igE antibodies are considered to recognize the primary structures of the peptides but not their comformational structures.

(2) Binding inhibiting assay

By performing primary screening, some peptides are obtained containing regions which are bound to IgE antibodies in the serum of a patient. Whether or not these peptides inhibit binding of patient's serum IgE antibodies with allergens is investigated using an enzyme antibody method similar to that described above.

Allergen solution is placed in a 96-well plate for coating, after which blocking is performed. Pooled sera from patients and a peptide having a predetermined concentration are added to each well and reaction is allowed to proceed for 4 hours at 37°C or over night at room temperature. Enzyme-labelled IgE antibodies are added to the plate after it has been washed. Reaction is allowed to proceed over night, and the plate is washed again. A substrate solution is added and reacted for 2 hours at 37°C, after which a reaction terminating solution is added. Absorbance is measured in each well using a fluorospectrophotometer.

(3) Histamine release inhibition test

Whether or not the peptide which inhibits binding of a patient's IgE antibody and an allergen suppresses formation of a cross-linking structure (which is indispensable to the onset of type I allergic disease) is investigated by a histamine release inhibition test using a patient's basophils. On basophils from an allergic patient, there are already IgE antibodies specific to allergen bound to the basophils. It is considered that peptides having monovalent epitopes corresponding to these IgE antibodies inhibit allergen from forming cross-linking structures when the peptides are bound to IgE antibodies, thereby inhibiting release of histamine from sensitized basophils. This inhibition test can be performed in the following manner. Haparinized peripheral blood collected from an allergic patient is reacted with a peptide, and then with an allergen. The resultant mixture is centrifugally separated, and the amount of free histamine in the supernatant is determined using, for example, a histamine kit "Eiken" manufactured by Eiken Kagaku K.K.

If fresh blood cannot be obtained from the patient to be examined, a passive histamine release inhibition test system may be used. Briefly, leukocytes in the blood collected from an allotype allergy subject in the presence of heparin are processed under acidic conditions so as to liberate IgE antibodies from basophils of the subject, after which the serum from the patient to be examined is added to the cells, thereby allowing the patient's IgE antibodies to be bound to IgE receptors on the basophils. Thus, basophils which have artificially been passively sensitized by serum IgE from an allergic subject can be obtained. Using such basophils, histamine release inhibition test can be performed.

If the entire primary structure of an allergen has not been elucidated, possible locations of B cell epitopes are narrowed down by reacting a single species or combinations of variety of proteases with an allergen for fragmentating the allergen into peptides with different lengths. Subsequently, the fragments are separated by, for example, SDS polyacrylamide gel electrophoresis (SDS-PAGE). Highly pure proteases are on the market. For example, a V8 protease, lysyl endopeptidase, chymotrypsin, subtilisin, and a thermolysin may be used for fragmentation of an allergen.

In order to identify a peptide containing B cell epitopes among the peptides on an SDS-PAGE gel, a Western blotting method may be used. Peptides on an SDS-PAGE gel are transfered onto a membrane, PVDF (polyvinylidene difluoride) using a semiblotter. After reacting with IgE antibodies in the patient's serum, a peptide containing B cell epitopes is identified by an enzyme antibody technique. A peptide which reacts with IgE antibodies in serum of the patient is extracted and purified, and its amino acid sequence is determined.

Whether or not a peptide which is bound to an IgE antibody also contains B cell epitopes which functionally participate to an allergic reaction is investigated using, as an index, potential of releasing histamine from basophils in peripheral blood of an allergy patient. A histamine release test may be performed using, for example, a histamine kit "Eiken" manufactured by Eiken Kagaku K.K. A peptide having histamine releasing capacity has at least two B cell epitopes which form a cross-linking structure by binding with IgE antibodies on mast cells or basophils. Based on the same idea as described above, fragmentary peptides covering the entire primary structure of the above peptide are synthesized with reference to the amino acid sequence of the peptide, thereby obtaining peptides each containing a corresponding monovalent epitope and inhibiting formation of a cross-linking structure. On this occasion, care is taken so that the fragmentary peptides share an adequate number of amino acids in an overlapping manner. Using the thus obtained fragmentary peptides, a histamine release inhibition test is performed based on the idea as described above.

The above-described anti-allergic agents according to the present invention can be used against a variety of allergens including food allergens such as ovalbumin and ovomucoid which are primary causal substances of egg allergy as well as αs1-casein and β-lactoglobulin which are causal substances of milk allergy; mite allergens (Der p I/Der f I and Der p II/Der f II) which are considered as the most important among vast allergens; and cryptomeria pollen allergens (Cry j I and Cryj II) which are causal substances of cryptomeria pollinosis.

Examples of peptides which are active components of the anti-allergic agent of the present invention include amino acid sequences selected from the following (1) through (17) and peptides having 3 or more residues of these amino acids.

(1) Glu-Phe-Arg-Ala-Asp-His-Pro-Phe-Leu-Phe,
(2) Lys-Ala-Ser-Ile-Asp-Gly-Leu-Glu-Val,
(3) Phe-Gln-Leu-Glu-Ala-Val-Phe-Glu-Ala,
(4) Ala-Asn-Gln-Asn-Thr-Lys-Thr-Ala-Lys,
(5) Lys-Ile-Glu-Ile-Lys-Ala-Ser-Ile-Asp,
(6) Ser-Ile-Asp-Gly-Leu-Glu-Val-Asp-Val,
(7) Lys-Cys-Pro-Leu-Val-Lys-Gly-Gln-Gln,
(8) Asn-Val-Pro-Lys-Ile-Ala-Pro-Lys-Ser,
(9) Pro-Lys-Ser-Glu-Asn-Val-Val-Val-Thr,
(10) Ser-Glu-Asn-Val-Val-Val-Thr-Val-Lys,
(11) Val-Val-Thr-Val-Lys-Val-Met-Gly-Asp,
(12) Ile-Ala-Thr-His-Ala-Lys-Ile-Arg-Asp,
(13) Gln-X-Lys-Trp-Val-Asn-Gly-Arg-Glu-Ile-X,
(14) Ser-Ala-Ser-Ala-X-Gln-Asn-Gln-Arg,
(15) X-Thr-Ser-Ala-Ser-Ala-X-Gln-Asn,
(16) Asn-Leu-Phe-Phe-Asn-Gly-Pro-X-Gln,
(17) Pro-Asn-X-Thr-Asn-Lys-X-His-Gly,

[wherein X represents Cys or an amino acid capable of being bound to an antibody].

The peptide (1) contains a monovalent B cell epitope of ovalbumin, peptides (2) through (12) each contain a monovalent B cell epitope of mite allergen Der p II, and peptides (13) through (17) each contain a monovalent B cell epitope of cryptomeria pollen allergen Cry j II. In addition, these peptides inhibit formation of a cross-linking structure occurable as a result of binding of respective allergens and IgE antibodies. Thus, these peptides inhibit release of histamine from sensitized basophils of corresponding allergy patients, and therefore, they are useful for preventing and treating allergic diseases caused by their counterpart allergens.

The peptides (1) through (17) are respectively composed of 9 to 11 amino acid residues. It is generally considered that in order for an amino acid residue to serve as a B cell epitope, 3 or more residues are needed. In view of this, peptides of 9 or less residues containing such amino acid sequences can function as monovalent B cell epitopes. Thus, peptides of 3 to 9 residues from these amino acid sequences may also be used.

When any of these peptides contain 1 to 2 residues of cysteine (Cys), this amino acid may be oxidized to produce a disulfide bond and to form a oligomer of peptides. Formation of an oligomer of peptides results in epitopes of multi-valency and not monovalent epitopes. Oxidized peptides resulting from epitopes of multi-valency form a cross-linking structure by themselves on sensitized cell surfaces, which is the second stage of the aforementioned allergic reaction. Therefore, peptides which may be used include such peptides in which cysteine (Cys) residues are replaced by other amino acids and having reactivity with an antibody.

The peptides of the present invention may be used singly. If different individuals show different specificities of a peptide, combinations of two or more peptides may be used.

The peptides of the present invention can be used as they are in the prevention and treatment of allergic diseases. If the peptides have sites at which enzymes in the tissue in the tissue act and decompose the peptides, it is also possible to use modified peptides in which monovalent epitope sites are remained but sites which are sensitive to the enzymes are replaced by other amino acids or chemical structures. Alternatively, those in which some of structural L- amino acids have been converted into D- amino acids may also be used.

When the peptide of the present invention is used in the prevention and treatment of allergic diseases, it is preferred that effective amounts of the peptide and pharmaceutically acceptable carriers are formed into a medicinal composition.

Examples of such carriers include vehicles, binders, lubricants, disintegrants, coating agents, emulsifiers, suspending agents, solvents, stabilizing agents, sorbent aids, and ointment bases. Compositions may be prepared into various forms for oral administration, injection administration, rectal administration, and external use.

Preferred formulations for oral administration include granules, tablets, sugar-coated tablets, capsules, pills, liquids, emulsions, and suspension; preferred formulations for injection administration include intravenous injection liquids, intermuscular injection liquids, subcutaneous injection liquids, and dripping liquids; preferred formulations for rectal administration include suppository soft capsules; and preferred formulations for external use include ointments, lotions, and liniments. Moreover, the compositions may also be prepared into eye drops and ear drops.

The amount of administration of the anti-allergic agent of the present invention differs depending on the manner of administration, symptom, body weight, etc. Generally, 1 to 1,000 mg/day is preferred.

Examples:

The present invention will next be described by way of examples. However, the present invention is not limited to these examples.

Example 1:

Anti-allergic agent for allergic diseases caused by egg allergens:

(1) (Identification of the binding region with patient's serum IgE)

Ovalbumin (OVA) and V8 protease were reacted at a ratio of 10:1, and SDS-polyacrylamide gel electrophoresis (SDS-PAGE) was performed. Using a semiblotter, enzyme-treated OVA fragments were subsequently transfered onto a PVDF membrane. Sera from patients suffering from egg allergy were respectively diluted to a 1:1.5 ratio and then allowed to react for 72 hours. Anti-OVA IgE antibodies were detected by an enzyme antibody technique. The amino acid sequence of the region where anti-OVA IgE antibodies were bound was analyzed using an Applied 377A Sequencer Analyzer. From this band, proteins were extracted. When the proteins were used in a histamine release test, they were extracted by the following method. First, a gel corresponding to the band was cut out and placed on a dialyzing membrane. A 80A steady current was passed through the membrane using a blotting buffer containing SDS, and proteins were extracted over a period of 5 days. Dialysis was further continued using PBS to remove SDS. As a result, 13 bands were detected at a molecular weight of 31000 or less. Although patients reacted differently, 8 out of 12 patients strongly reacted with the band having the smallest molecular weight. Thus, this band was extracted, and its amino acid sequence was determined. Consequently, it was found that the binding region with IgE antibodies in the primary sequence of OVA corresponded to $OVA_{347-385}$ in a C-terminal (Fig. 1).

(2) (Histamine release caused by enzyme-lysed OVA fragments)

In order to confirm that the region $OVA_{347-385}$ found to be the binding region for anti-OVA IgE antibodies also participates to allergic reactions, a histamine release test was performed using $OVA_{347-385}$ fragments.

The histamine release test was performed using a histamine kit "Eiken" manufactured by Eiken Kagaku K.K. Briefly, heparinized peripheral blood was collected from each patient, and the blood was diluted to a 1:3 ratio. Fifty µl of an antigen liquid which had been diluted with a buffer for release tests and 100 μl of the 1:3 diluted blood were mixed and reacted for 30 minutes at 37°C. The reaction liquid was then centrifugally separated at 4°C for 5 minutes and the supernatant was used as a test sample. One hundred μl of the test sample and 50 μl of an acylating buffer were added in an acylating test tube so as to cause reaction. One ml of $^{125}$I-labelled histamine was further added thereto. The content was dispensed into histamine antibody tubes, each 500 μl. After the histamine tubes were incubated for 16 to 24 hours, the solution was drawn off using an aspirator, and measured using a gamma scintillation counter. The amount of total histamine was determined using a supernatant sample obtained by adding 1 ml of purified water to 50 μl of whole blood, freezing and thawing, then centrifugally separating. Results were processed in the following manner. The amount of histamine release was obtained from the standard curve of histamine. The amount of histamine in a control sample in which only buffer was reacted was subtracted from this amount. The ratio of the difference with respect to the total amount of histamine was calculated and taken as a histamine release ratio. When untreated OVA was used to stimulate basophils from patients suffering from egg allergy, histamine was released in a concentration dependent manner. Similarly, when OVA fragments were used for stimulation, histamine was released in a concentration dependent manner. On the other hand, in a test using basophils from healthy subjects, release of histamine was not observed in either case where OVA or OVA fragments were used. Accordingly, it was made clear that at least two epitopes are present on $OVA_{347-385}$, and they are bound to IgE antibodies on surfaces of patients' basophils, causing cross-linking and aggregate IgE receptors.

(3) (Histamine release using synthesized peptides)

Using a peptide synthesizer, peptides were synthesized by fragmenting the region corresponding to OVA $_{347-385}$ into peptide fragments each having 20 residues with reference to the amino acid sequence of OVA in Fig. 1. The synthesized peptides were arranged so that 10 residues were shared by any two of the thus-prepared fragmentary peptides. Thus, 3 kinds of peptides, peptide A ($OVA_{347-366}$), peptide B ($OVA_{357-376}$), and peptide C ($OVA_{367-385}$) were synthesized.

In a manner similar to that described in (2) above, basophils from patients were stimulated with untreated OVA or with each synthesized peptide, and the site of the peptide at which histamine was released was examined. In cases of egg allergy patients, when their basophils were stimulated with untreated OVA, release of histamine was clearly observed. Separately, when their basophils were stimulated with peptide B and peptide C, histamine was released in a concentration dependent manner. However, stimulation with peptide A did not cause release Of histamine. From these results, it was considered that peptides B and C each have 2 or more epitopes for IgE antibodies. Peptide A was suggested to have no antigen determinant or only one antigen determinant, if any. In view of the fact that histamine release was not observed in healthy subjects stimulated with any of peptides A, B, or C or with untreated OVA, it was confirmed that the release of histamine caused by these peptides was not a non-specific reaction.

(4) (Histamine release inhibition by synthesized peptides)

According to the amino acid sequences of peptides A and B, a peptide D ($OVA_{357-366}$) was synthesized using a peptide synthesizer.

Peptide D was examined as to whether it could inhibit release of histamine due to peptide B or untreated OVA.

The histamine release inhibition test was performed using a histamine kit "Eiken" manufactured by Eiken Kagaku K.K. Briefly, heparinized peripheral blood was collected from each patient, and the blood was diluted to a 1:1 ratio using a buffer for release tests. Equivalent amounts of a diluted peptide D (which had been diluted to double the density of an inhibitory concentration using a buffer for release tests) and a diluted blood from a patient (which had been diluted to a 1:1 ratio) were mixed and allowed to react overnight at 4°C. Subsequently, fifty μl of peptide B or OVA diluted with a buffer for release was mixed with 100 μl of the patient's blood with which peptide D was reacted. The mixture was allowed to react for 30 minutes at 37°C. The reaction liquid was then centrifugally separated at 4°C for 5 minutes and the supernatant was used as a test sample. One hundred μl of the test sample and 50 μl of an acylating buffer were added in an acylating test tube so as to cause reaction. One ml of $^{125}$I-labelled histamine was further added thereto. The content was dispensed into histamine antibody tubes, each 500 μl. After the histamine tubes were incubated for 16 to 24 hours, the solution was drawn off using an aspirator, and measured using a gamma scintillation counter. The amount of total histamine was determined using a supernatant sample obtained by adding 1 ml of purified water to 50 μl of whole blood, freezing and thawing, then centrifugally separating. Results were processed in the following manner. The amount of histamine release was obtained from the standard curve of histamine. The amount of histamine in a control sample in which only buffer was reacted was subtracted from this amount. The ratio of the difference with respect to the total amount of histamine was calculated and taken as a histamine release ratio. Inhibition ratio (%) was calculated by the following equation:

%Inhibition = {1-(histamine release ratio in the presence of an inhibitory peptide)/(histamine release ratio in the absence of an inhibitory peptide)} x 100

The results are shown in Figs. 2 and 3.

The histamine release caused by a stimulant peptide B was suppressed by a peptide D in a concentration dependent manner, and peptide D at a concentration of not less than 1.4 mg/ml exhibited at least 85% of suppression. On the other hand, the histamine release caused by an untreated OVA was suppressed by a peptide D (1.4 mg/ml) with a suppression ratio of 93%. From these results, it is understood that histamine is released from basophils of a patient by a stimulant peptide B or untreated OVA. However, when peptides D occupy the antigen binding sites of IgE antibodies, IgE antibodies are prevented from being cross-linked even though stimulant peptides B or untreated OVAs are later bound to another set of IgE antibodies on the patient's basophils. Thus, signals of histamine release are not transmitted, inhibiting histamine to be released.

Example 2:

Anti-allergic agent for allergic diseases caused by mite allergens:

(1) (Synthesis of overlapping peptides)

The complete primary structure of the amino acid sequence of a Der p II protein was determined after cDNA was isolated therefrom by Chua et al. (Int. Arch. Allergy Appl. Immunol. 91: 118-123, 1990). Based on the amino acid sequence, peptides were synthesized on a solid phase using a multipin system (manufactured by Chiron) and coupling reagents, Fmoc-amino acid and DCC (dicyclohexyl carbodiimide) (Geysen, H.M. et al., Proc. Natl. Acad. Sci. USA. 81:3998-4002, 1984). Sixty-one kinds of peptides were synthesized, the first peptide comprising the first through ninth amino acids of the N-terminal of Der p II, the second peptide comprising the third through eleventh amino acids of the N-terminal, and the third through 61st peptides being prepared in an analogous manner.

(2) (Identification of synthesized peptides which are bound to serum IgE of patients)

Sera of mite rhinallergosis patients were intravenously collected from 15 patients demonstrating severe rhinitis conditions. The RAST (radio-allergosorbent test, Pharmacia) values as determined using antigens from two mite species, Dermatophagoides pteronyssinus (Der p) and Dermatophagoides farina (D f), were class 4. Each of the above-mentioned 61 synthesized peptides and 100 µl of each patient's serum were placed in a 96-well plate and reacted overnight at room temperature. Synthesized peptides in a solid phase were washed three times to remove the patient's serum using a washing solution of 0.05% Tween 20/0.15 M NaCl/Tris-HCl (pH 7.5). 100 µl of galactosidase-labelled anti-human IgE antibodies (Pharmacia) were diluted to 40-fold using a 1:9 diluted Block-ace (product of Dainippon Seiyaku K.K.). The diluted solution was added to a 96-well plate, each synthesized solid phase peptide was also added, and reaction, was allowed to proceed overnight at room temperature. The synthesized solid phase peptide was washed with the same washing solution five times. Thereafter, to a 96-well black plate (manufactured by Dainippon Seiyaku K.K.), were added 200 µl of a solution of 0.1 mM 4-methylumbelliferyl-β-D-galactopyranoside in 0.1% bovine serum albumin/0.1 M NaCl/1 mM $MgCl_2$/0.1% sodium azide/10 mM phosphate buffer (pH 7.0). Subsequently, the synthesized solid phase peptide was added and reacted for 2 hours at 37°C. After completion of reaction, 100 µl of a reaction terminating solution (0.1 M glycin/NaOH, pH 10.3) were added to each well, and absorbance was measured using a fluorospectrophotometer for 96-well plates (Manufactured by Flow). This reaction was studied for each of 15 patients.

Upon studying reactions between serum from each of 15 patients suffering from rhinallergosis caused by mites and 61 kinds of overlapping peptides (solid phase peptides) which entirely cover a Der p II protein molecule, it was found that the following peptides exhibited binding reactions with IgE antibodies (Fig. 4).

Lys-Ala-Ser-Ile-Asp-Gly-Leu-Glu-Val (synthesized peptide No. 28; 55-63)
Phe-Gln-Leu-Glu-Ala-Val-Phe-Glu-Ala (synthesized peptide No. 18; 35-43)
Ala-Asn-Gln-Asn-Thr-Lys-Thr-Ala-Lys (synthesized peptide No. 22; 43-51)
Lys-Ile-Glu-Ile-Lys-Ala-Ser-Ile-Asp (synthesized peptide No. 26; 51-59)
Ser-Ile-Asp-Gly-Leu-Glu-Val-Asp-Val (synthesized peptide No. 29; 57-65)
Lys-Cys-Pro-Leu-Val-Lys-Gly-Gln-Gln (synthesized peptide No. 39; 77-85)
Asn-Val-Pro-Lys-Ile-Ala-Pro-Lys-Ser (synthesized peptide No. 47; 93-101)
Pro-Lys-Ser-Glu-Asn-Val-Val-Val-Thr (synthesized peptide No. 50; 99-107)
Ser-Glu-Asn-Val-Val-Val-Thr-Val-Lys (synthesized peptide No. 51; 101-109)
Val-Val-Thr-Val-Lys-Val-Met-Gly-Asp (synthesized peptide No. 53; 105-113)
Ile-Ala-Thr-His-Ala-Lys-Ile-Arg-Asp (synthesized peptide No. 61; 121-129)

Accordingly, it was made clear that there are IgE antibodies which recognize the primary structure of a Der p II protein in serum of a patient suffering from rhinallergosis caused by mites. From this result, it was suggested that these peptides potentially inhibit the binding reaction between IgE antibodies and a Der p II protein.

(3) (Inhibition of binding by a synthesized peptide (55-63))

A crude mite body extract (MBE) was prepared from a mite, Dermatophagoides pteronyssinus (Der p), by homogenizing Der p mite bodies in 0.15 M NaCl/phosphate buffer (pH 7.3, PBS) using a Teflon homogenizer and centrifugally separating to collect the supernatant. A pooled serum IgE from patients was prepared by blending sera from 10 patients suffering from rhinallergosis caused by mites (RAST values with respect to Dermat ophagoides pteronyssinus: 3 or more). The peptide (Der p II 55-63) was synthesized by a Fmoc method using a peptide synthesizer pssm 8 manufactured by Shimadzu Corporation. The results of the synthesis were confirmed by an amino acid analysis and by an analysis of the primary structure. The MBE was added to each well of a 96-well black plate (Dainippon Seiyaku k.K.) so that the amount of protein contained was 1 µg/well (100 µl). The plate was allowed to stand overnight at 4°C. After removing the MBE solution from each well, to each well were added 100 µl of a solution obtained by mixing a predetermined concentration of a peptide Der p II 55-63 and the pooled serum from patients at a ratio of 1:1. The plate was then allowed to stand overnight at room temperature. The solution in each well was decanted, and the wells were washed with a washing solution three times. To each well were added 100 µl of a solution obtained by diluting to 40-fold galactosidase-labelled anti-human IgE antibodies (Pharmacia) using a 10-fold diluted Block-ace (manufactured by Dainippon Seiyaku K.K.). Reaction was allowed to proceed over night at room temperature. The solution was removed from each well, and the wells were washed with the above-mentioned washing solution five times. Thereafter, to each well were added 200 µl of a solution of 0.1 mM 4-methylumbellylpheryl-β-D-galactopyranoside in 0.1% bovine serum albumin/0.1 M NaCl/1 mM $MgCl_2$/0.1% sodium azide/10 mM phosphate buffer (pH 7.0). Reaction was allowed to proceed for 2 hours at 37°C. After completion of reaction, 100 µl of a reaction terminating liquid (0.1 M glycin/NaOH, pH 10.3) were added to each well, and absorbance was measured using a fluorospectrophotometer for 96-well plates (Manufactured by Flow). The absorbance in a control which was prepared by adding a PBS solution in place of the peptide was taken as 100%.

An experiment was performed by newly synthesizing a Der p II peptide (55-63) which is soluble in PBS and by using a pooled serum of 10 patients suffering from rhinallergosis caused by mites. The object of the experiment was to determined how the peptide affects the binding reaction with an MBE, a crude extract of Der p mite bodies (Fig. 5). It was found that, binding of the pooled patients' serum IgE to MBE was inhibited by 20% with the peptide concentration of about 5 µg/ml - 5 mg/ml. Since a pooled serum from 10 patients suffering from rhinallergosis caused by mites was used in this experiment, it was concluded that Der p II peptide (55-63) serves as an epitope of IgE in many patients suffering from rhinallergosis caused by mites.

(4) (Histamine release inhibition by a synthesized peptide (55-63))

To 20 ml of heparinized venous blood, 5 ml of a 4.5% dextran solution were added and the mixture was allowed to stand for 30 to 60 minutes at room temperature. The upper leukocyte layer was extracted and subjected to a repetitive centrifugal operation. The leukocytes were then washed with PBS. To the leuokocytes in a precipitated fraction, 20 ml of a lactate buffer (pH 3.9, 4°C) were added and thoroughly mixed. After cooling the mixture on ice for 2 to 5 minutes, the pH was returned to neutral by adding PBS (25 ml). By this operation, IgEs on basophils were released. The leukocytes were washed well with PBS and suspended in 0.5% human albumin/PBS. 100 µl of cell suspension (approximately 2 x $10^5$ cells/well) were added to a V-bottom 96-well plate. Fifty µl of a pooled serum of patients suffering from rhinallergosis caused by mites were further added, and reaction was allowed to proceed for 1 hour at 37°C. This procedure caused serum IgEs from the patients suffering from rhinallergosis caused by mites to be bound to IgE receptors. Thus, basophils artificially sensitized in a passive manner with serum IgEs from patients suffering from rhinallergosis caused by mites can be obtained (Pruzansky J.J. et al., J. Immunol., 131; 1949-1953, 1983). The plate was centrifuged to remove 100 µl of a supernatant. 100 µl of a 0.5% human albumin/PBS were added to the residue and centrifuged again. The centrifugal operation was repeated twice, and the cells were then washed. 100 µl of a Der p II peptide (55-63) having a predetermined concentration of PBS were combined with 50 µl of an MBE (10 µg/50 µl) or PBS, and allowed to react for 30 minutes at 37°C. The plate was then centrifuged to collect 100 µl of a supernatant. The amount of histamine was determined using a histamine assay kit manufactured by Eiken Kagaku K.K.

Essentially, a histamine release test is performed to determine the amount of histamine released when an antigen solution (an allergen solution) is added to a sample of whole blood (fresh blood) from an allergic patient. However, if fresh blood cannot be obtained from the patient, a passive experimentation may be performed. Briefly, this experimentation system can be described as follows: From basophils in blood of allergic patients who react with allergens other than mite allergens, e.g., from patients suffering from cryptomeria pollen allergy, IgE antibodies which are reactive with cryptomeria are liberated, after which serum IgE from a patient suffering from rhinallergosis caused by mites is added. As a result, IgE antibodies specific to a mite allergen are bound to IgE receptors on basophils, thereby artificially causing histamine to be released in the presence of mite allergens in a manner analogous to the case where blood from patients suffering from rhinallergosis caused by mites was used (Pruzansky J.J. et al., J. Immunol., 131; 1949-1953, 1983). Using this experimental system, a study was performed as to whether a Der p II peptide (55-63) serves as a monovalent epitope and whether or not it can suppress release of histamine from basophils due to mite allergens (Fig. 6) if the peptide serves as a monovalent epitope. Firstly, it was confirmed that histamine was not released in a control system in which PBS was used (an antigen MBE was not added), and that histamine was released when an MBE (a crude extract of Der p mite bodies) was added. In a system in which only a Der p II peptide (55-63) was added (an MBE was not added), histamine was scarcely released (white bars in Fig. 6). By contrast, when an MBE was added in the presence of a Der p II peptide (55-63), the peptide suppressed histamine release in an amount of about 90% at a concentration of 7.55 mg/ml though it could not suppress histamine release at low concentrations. From these results, it was concluded that a Der p II peptide (55-63) is a monovalent epitope and is capable of suppressing histamine release attributed to mite allergens.

Reference Example:

Expression of cryptomeria pollen allergen Cry j II

(1) Collection of cryptomeria pollens:

Pollens of cryptomeria were collected from male flowers blossomed on sprays cut in February in Shizuoka and Kanagawa Prefectures. The cryptomeria pollens to be used for preparing Cry j I antigens were stored at -70°C, and those for preparing RNA were rapidly frozen in liquid nitrogen and then stored at -70°C.

(2) Extraction of RNA

RNA was extracted from cryptomeria pollens according to a method of Breiteneder et al. (Int. Arch. Allergy Appl. Immunol. 87:19-24, 1988) with some modifications.

One gram of cryptomeria pollens stored in a frozen state was suspended in 15 ml of an ice-cooled buffer for extraction (100 mM LiCl, 10 mM $Na_2EDTA$, 1% SDS, 20% 2-mercaptoethanol, and 100 mM Tris-HCl, pH 9.0). Further, 15 ml of phenol : chloroform : isoamyl alcohol (24:24:1) were added to the suspension. The resulting suspension was transferred to a homogenizer made of Teflon and homogenized with 20-30 strokes while rotating a Teflon pestle by a motor at the maximum speed. Subsequently, the homogeneous system was centrifugally separated into two layers; an aqueous layer and an organic layer (10,000 g, 15 min). The aqueous layer was taken, to which was added the same amount of a mixture, phenol : chloroform : isoamyl alcohol (24:24:1). The resulting mixture was shaken for 5 minutes and then centrifuged (10,000 g, 15 min) to obtain an aqueous layer. This same procedure was repeated twice. Then, 15 ml of a mixture, chloroform : isoamyl alcohol (24:1), was added and the same procedure was performed once. The resultant aqueous layer was combined with the same amount of 4 M LiCl and allowed to stand overnight at -20°C. The frozen solution was thawed at room temperature, and a pellet was obtained by centrifugation (20,000 g, 30 min). The pellet was dissolved in a small amount of sterilized distilled water, and the solution was combined with 0.3 volumes of 3M $CH_3COONa$ (pH 5.2) and 2.5 volumes of ethanol, and then allowed to stand for 60 minutes at -20°C. Sediments collected by centrifugation (10,000 g, 30 min) were re-dissolved in sterilized distilled water. Whole RNA fractions were thus prepared.

(3) Preparation of cryptomeria pollen mRNA and synthesis of cDNA

One mg of the whole RNA from cryptomeria pollens was used as a starting material, to which was added the same amount of a binding buffer (3M NaCl, 1 mM EDTA, 10 mM Tris-HCl, pH 7.4). The mixture was adsorbed onto oligo-dT-cellulose placed in a span column (manufactured by Clonetech Laboratories Inc., CA, USA), and eluted with a buffer for elution (1 mM EDTA, 10 mM Tris-HCl, pH 7.4), thereby obtaining approximately 10 µg of purified mRNA (the protocol attached to the column of Clonetech Lab. Inc. was followed). Subsequently, approximately 4 µg of cDNA were synthesized from 5 µg of the purified mRNA using a cDNA Synthesis System Plus (manufactured by Amersham International Plc., Buckinghamshare, England) according to the attached protocol.

(4) Synthesis of an oligonucleotide probe

The amino acid sequence of 10 residues from the N-terminal of Cry j II is shown in Fig. 7(A). The sequences of cDNA speculated from this amino acid sequence are shown in Fig. 7(B). Oligonucleotide probes (Oligo CJII) complementary to these sequences were synthesized as mixtures of 16 probes since two different bases were used at 4 locations (Fig. 7(C)). In order to decrease the number of types of the mixtures, G:T base pairs were permitted.

(5) Cloning of Cry j II cDNA

Using a cDNA cloning system lambda-gt10 (manufactured by Amersham International Plc., Buckinghamshare, England), a cDNA library was made according to the protocol attached to the kit. One µg of the above-described cDNA was integrated into the lambda-gt10 and a cDNA library was constructed. From the library having a size of about 500,000, about 5,000 clones were taken and placed on a plate having a diameter of 150 mm. The probe for screening was prepared by labelling the aforementioned oligonucleotide (Oligo CJII) with a T4 polynucleotide kinase using a [gamma-$^{32}$P]ATP (7,000 Ci/mmol manufactured by ICN Biochemicals. Inc.). A nitrocellulose filter to which phage DNA was fixed was soaked in a solution containing 5xSSPE (1xSSPE: 0.18 M NaCl, 10 mM sodium phosphate, 1 mM EDTA), 5xFBP (1xFBP: 0.02% Ficoll, 0.02% bovine serum albumin, 0.02% polyvinylpyrrolidone), 0.3% SDS and 100 µg/ml tRNA for at least 1 hour at 48°C, thereby causing hybridization. Thereafter, the nitrocellulose filter was soaked in the same but newly prepared solution, to which was added a probe labelled with $^{32}$P (Oligo CJII). Hybridization was allowed to proceed overnight at 48°C. The filter was then washed with a solution containing 6xSCC (1xSCC: 0.15 M NaCl, 0.015M sodium citrate) and 0.1% SDS for 5 minutes at room temperature (30°C). The washed filter was next subjected to autoradiography. Four strong signals were detected. Phage DNA was extracted from one of them, and cleaved with a restriction enzyme EcoRI. As a result, it was found that a DNA fragment of about 1.7 Kbp had been inserted. The inserted fragment was subcloned with pUC118, and a deletion mutant was prepared using a kilosequence deletion kit (manufactured by Takara Shuzo Co., Ltd.). The resulting deletion mutant was used to determine the complete nucleotide sequence. The nucleotide sequence was determined by first causing a primer elongation reaction using a synthesized primer and a chromogen-labelled dideoxyterminator and then reading by an automated sequencer (model 370A, Applied Biosystems, Japan). The results are shown as Sequence No. 5. In this sequence, the nucleotide sequence of the DNA in the translation region is shown as Sequence No. 4, and the amino acid sequence predicted from this nucleotide sequence is shown as Sequence No. 2. In this nucleotide sequence, the nucleotide sequence of Cry j II predicted from the amino acid sequence of the N-terminal of mature Cry j II is shown as Sequence No. 3, and the amino acid sequence predicted from this nucleotide sequence is shown as Sequence No. 1.

(6) Expression of Cry j II cDNA in *E. Coli*

The *E. Coli* expression vector pGEMEX-1 commercially sold by Promega has a T7 promoter, a coding sequence for T7 gene 10, and a T7 terminator, and achieves high expression by introducing an open reading frame inserted into a multi-cloning site located downstream of T7 gene 10 into an E. Coli capable of expressing T7 RNA polymerase (e.g., BL21(DE3)). The aforementioned Cry j II cDNA was digested with BamHI (the adapters ligated to both ends of cDNA contain BamHI sites), and cDNA fragments were cut out. Each of them was integrated into a BamHI site of pGEMEX-1, thereby constructing a Cry j II expression vector pEXCJII. pEXCII can express a fusion protein (T7 Cry j II, 73 kD) of a T7 gene 10 product (23 kD) and a Cry j II protein (50 kD). The transformant prepared by introducing pEXCII into an E. Coli, BL21(DE3), was cultured. Using IPTG, T7 RNA polymerase was urged to express Cry j II. The cell extract of the expressed *E. Coli* was subjected to SDS polyacrylamide gel electrophoresis. As a result, bands at about 73 kD were observed on the pEXCII-holding BL21(DE3) which were considered to be attributed to T7 Cry j II. However, in BL21(DE3) which held a control pGEMEX-1 and in the parent strain BL21(DE3), these bands were not observed.

Reactivity of a fusion protein (T7 Cry j II) of Cry j II and T7 gene 10 and serum from a patient suffering from cryptomeria pollinosis: An extract of *E. Coli* which had expressed T7 Cry j II was subjected to electrophoresis on an SDS polyacrylamide gel, and then to Western blotting on a PVDF membrane manufactured by Millipore. Thus, reactivity with sera from 5 pollinosis patients and 3 healthy subjects was studied. As controls, BL21 extracts which had expressed pGEMEX-1-holding BL21 and a fusion protein (T7 Cry j I) of T7 gene 10 and Cry j I and native Cry j I purified from cryptomeria pollens were simultaneously blotted and observed for their reactions. As shown in Fig. 8, 2 patients' sera reacted with T7 Cry j II. Both sera reacted with T7 Cry j II and with native Cry j I, but did not react with pGEMEX-1 harboring Bl 21 extract or T7 Cry j I. From these results, it was confirmed that T7 Cry j II had antigenity causing reactions with serum IgE of cryptomeria pollinosis patients.

Example 3:

Anti-allergic agent against allergic diseases caused by cryptomeria pollen allergens

A Cry j II allergen protein, as known from the deduced primary structure of amino acids which starts with an initiating codon (Met) from cDNA, consists of 514 amino acid residues (Sequence No. 2). However, in view that the N-terminal amino acids of Cry j II derived from cryptomeria pollens are reported as Ala-Ile-Asn (Sakaguchi, M., et al. Allergy 45, 309-312. 1990), the N-terminal moiety is considered to be decomposed in naturally occurring actual Cry j II. Moreover, from the molecular weight of Cry j II derived from cryptomeria pollens, the C-terminal moiety is also presumed to be partially decomposed. Presently, however, the C-terminal amino acids are unknown. Therefore, overlapping peptides were synthesized on a solid phase using an Fmoc-amino acid and multipin system (manufactured by Chiron) so as to cover all the proteins consisting of 460 amino acid residues (Sequence No. 1) other than the N-terminal moiety. The first peptide comprises the first through ninth amino acids in the N-terminal moiety of a Cry j II protein. The second peptide comprises the third through eleventh amino acids in the N-terminal moiety. Likewise, 227 kinds of peptides in total were synthesized.

Sera of patients suffering from cryptomeria pollinosis were collected from patients who demonstrated severe allergic conjunctivitis or allergic rhinitis from February to April and who had RAST (radio-allergosorbent test, Pharmacia) values of at least class 3 induced by cryptomeria pollens. 227 kinds of peptides synthesized as described above and 100 µl of each patient's serum were reacted in a 96-well plate overnight at room temperature. Subsequently, using a washing solution of 0.05% Tween 20/0.15 M NaCl/Tris-HCl (pH 7.5), synthesized solid phase peptides were washed three times to remove the patient's serum. Using a 1:9 diluted Block-ace (product of Dainippon Seiyaku K.K.), 100 µl of galactosidase-labelled anti-human IgE antibodies (Pharmacia) were diluted to 40-fold. The diluted solution was added to a 96-well plate, and then each synthesized solid phase peptide was also added. Reaction was allowed to proceed overnight at room temperature. The synthesized solid phase peptide was washed with the same washing solution five times. Thereafter, 200 µl of a solution of 0.1 mM 4-methylumbelliferyl-β-D-galactopyranoside in 0.1% bovine serum albumin/0.1 M NaCl/1 mM $MgCl_2$/0.1% sodium azide/10 mM phosphate buffer (pH 7.0) were added to a 96-well black plate (manufactured by Dainippon Seiyaku K.K.). Subsequently, the synthesized solid phase peptide was added and reacted for 2 hours at 37°C. After completion of reaction, 100 µl of a reaction terminating liquid (0.1 M glycin/NaOH, pH 10.3) were added to each well, and absorbance was measured using a fluorospectrophotometer for 96-well plates (Manufactured by Flow).

As a result, the following peptides reacted serum IgE of cryptomeria pollinosis patients and thus they were confirmed to be B-cell epitopes.

Gln-Cys-Lys-Trp-Val-Asn-Gly-Arg-Glu,
Lys-Trp-Val-Asn-Gly-Arg-Glu-Ile-Cys,
Ser-Ala-Ser-Ala-Cys-Gln-Asn-Gln-Arg,
Cys-Thr-Ser-Ala-Ser-Ala-Cys-Gln-Asn,
Asn-Leu-Phe-Phe-Asn-Gly-Pro-Cys-Gln,
Pro-Asn-Cys-Thr-Asn-Lys-Cys-His-Gly.

Industrial Applicability:

The anti-allergic agents of the present invention are based on a completely new mechanism of inhibiting the cross-linking phenomenon of IgE antibodies in an antigen specific manner. The agents are useful in the prevention and treatment of immediate allergies such as food allergies, mite allergies, bronchial asthma, pollinosis, allergic rhinitis, and allergic enterogastritis.

Sequence Listing

Sequence No. 1

Length of the sequence: 460

Type of the sequence: amino acid

Topology: linear

Kind of the sequence: peptide

Sequence

```
Ala Ile Asn Ile Phe Asn Val Glu Lys Tyr Gly Ala Val Gly Asp Gly
                    5                  10                  15
Lys His Asp Cys Thr Glu Ala Phe Ser Thr Ala Trp Gln Ala Ala Cys
                20                  25                  30
Lys Asn Pro Ser Ala Met Leu Leu Val Pro Gly Ser Lys Lys Phe Val
            35                  40                  45
Val Asn Asn Leu Phe Phe Asn Gly Pro Cys Gln Pro His Phe Thr Phe
        50                  55                  60
Lys Val Asp Gly Ile Ile Ala Ala Tyr Gln Asn Pro Ala Ser Trp Lys
65                  70                  75                  80
Asn Asn Arg Ile Trp Leu Gln Phe Ala Lys Leu Thr Gly Phe Thr Leu
                    85                  90                  95
Met Gly Lys Gly Val Ile Asp Gly Gln Gly Lys Gln Trp Trp Ala Gly
                100                 105                 110
Gln Cys Lys Trp Val Asn Gly Arg Glu Ile Cys Asn Asp Arg Asp Arg
            115                 120                 125
Pro Thr Ala Ile Lys Phe Asp Phe Ser Thr Gly Leu Ile Ile Gln Gly
        130                 135                 140
Leu Lys Leu Met Asn Ser Pro Glu Phe His Leu Val Phe Gly Asn Cys
145                 150                 155                 160
Glu Gly Val Lys Ile Ile Gly Ile Ser Ile Thr Ala Pro Arg Asp Ser
                    165                 170                 175
```

Pro Asn Thr Asp Gly Ile Asp Ile Phe Ala Ser Lys Asn Phe His Leu
180 185 190
Gln Lys Asn Thr Ile Gly Thr Gly Asp Asp Cys Val Ala Ile Gly Thr
195 200 205
Gly Ser Ser Asn Ile Val Ile Glu Asp Leu Ile Cys Gly Pro Gly His
210 215 220
Gly Ile Ser Ile Gly Ser Leu Gly Arg Glu Asn Ser Arg Ala Glu Val
225 230 235 240
Ser Tyr Val His Val Asn Gly Ala Lys Phe Ile Asp Thr Gln Asn Gly
245 250 255
Leu Arg Ile Lys Thr Trp Gln Gly Gly Ser Gly Met Ala Ser His Ile
260 265 270
Ile Tyr Glu Asn Val Glu Met Ile Asn Ser Glu Asn Pro Ile Leu Ile
275 280 285
Asn Gln Phe Tyr Cys Thr Ser Ala Ser Ala Cys Gln Asn Gln Arg Ser
290 295 300
Ala Val Gln Ile Gln Asp Val Thr Tyr Lys Asn Ile Arg Gly Thr Ser
305 310 315 320
Ala Thr Ala Ala Ala Ile Gln Leu Lys Cys Ser Asp Ser Met Pro Cys
325 330 335
Lys Asp Ile Lys Leu Ser Asp Ile Ser Leu Lys Leu Thr Ser Gly Lys
340 345 350
Ile Ala Ser Cys Leu Asn Asp Asn Ala Asn Gly Tyr Phe Ser Gly His
355 360 365
Val Ile Pro Ala Cys Lys Asn Leu Ser Pro Ser Ala Lys Arg Lys Glu
370 375 380
Ser Lys Ser His Lys His Pro Lys Thr Val Met Val Glu Asn Met Arg
385 390 395 400
Ala Tyr Asp Lys Gly Asn Arg Thr Arg Ile Leu Leu Gly Ser Arg Pro

405 410 415

Pro Asn Cys Thr Asn Lys Cys His Gly Cys Ser Pro Cys Lys Ala Lys

420 425 430

Leu Val Ile Val His Arg Ile Met Pro Gln Glu Tyr Tyr Pro Gln Arg

435 440 445

Trp Ile Cys Ser Cys His Gly Lys Ile Tyr His Pro

450 455 460

Sequence No. 2

Length of the sequence: 514

Type of the sequence: amino acid

Topology: linear

Kind of the sequence: peptide

Sequence

Met Ala Met Lys Leu Ile Ala Pro Met Ala Phe Leu Ala Met Gln Leu

5 10 15

Ile Ile Met Ala Ala Ala Glu Asp Gln Ser Ala Gln Ile Met Leu Asp

20 25 30

Ser Val Val Glu Lys Tyr Leu Arg Ser Asn Arg Ser Leu Arg Lys Val

35 40 45

Glu His Ser Arg His Asp Ala Ile Asn Ile Phe Asn Val Glu Lys Tyr

50 55 60

Gly Ala Val Gly Asp Gly Lys His Asp Cys Thr Glu Ala Phe Ser Thr

65 70 75 80

Ala Trp Gln Ala Ala Cys Lys Asn Pro Ser Ala Met Leu Leu Val Pro

85 90 95

Gly Ser Lys Lys Phe Val Val Asn Asn Leu Phe Phe Asn Gly Pro Cys

100 105 110

Gln Pro His Phe Thr Phe Lys Val Asp Gly Ile Ile Ala Ala Tyr Gln
115 120 125

Asn Pro Ala Ser Trp Lys Asn Asn Arg Ile Trp Leu Gln Phe Ala Lys
130 135 140

Leu Thr Gly Phe Thr Leu Met Gly Lys Gly Val Ile Asp Gly Gln Gly
145 150 155 160

Lys Gln Trp Trp Ala Gly Gln Cys Lys Trp Val Asn Gly Arg Glu Ile
165 170 175

Cys Asn Asp Arg Asp Arg Pro Thr Ala Ile Lys Phe Asp Phe Ser Thr
180 185 190

Gly Leu Ile Ile Gln Gly Leu Lys Leu Met Asn Ser Pro Glu Phe His
195 200 205

Leu Val Phe Gly Asn Cys Glu Gly Val Lys Ile Ile Gly Ile Ser Ile
210 215 220

Thr Ala Pro Arg Asp Ser Pro Asn Thr Asp Gly Ile Asp Ile Phe Ala
225 230 235 240

Ser Lys Asn Phe His Leu Gln Lys Asn Thr Ile Gly Thr Gly Asp Asp
245 250 255

Cys Val Ala Ile Gly Thr Gly Ser Ser Asn Ile Val Ile Glu Asp Leu
260 265 270

Ile Cys Gly Pro Gly His Gly Ile Ser Ile Gly Ser Leu Gly Arg Glu
275 280 285

Asn Ser Arg Ala Glu Val Ser Tyr Val His Val Asn Gly Ala Lys Phe
290 295 300

Ile Asp Thr Gln Asn Gly Leu Arg Ile Lys Thr Trp Gln Gly Gly Ser
305 310 315 320

Gly Met Ala Ser His Ile Ile Tyr Glu Asn Val Glu Met Ile Asn Ser
325 330 335

Glu Asn Pro Ile Leu Ile Asn Gln Phe Tyr Cys Thr Ser Ala Ser Ala
340 345 350

Cys Gln Asn Gln Arg Ser Ala Val Gln Ile Gln Asp Val Thr Tyr Lys
355 360 365

Asn Ile Arg Gly Thr Ser Ala Thr Ala Ala Ala Ile Gln Leu Lys Cys
370 375 380

Ser Asp Ser Met Pro Cys Lys Asp Ile Lys Leu Ser Asp Ile Ser Leu
385 390 395 400

Lys Leu Thr Ser Gly Lys Ile Ala Ser Cys Leu Asn Asp Asn Ala Asn
405 410 415

Gly Tyr Phe Ser Gly His Val Ile Pro Ala Cys Lys Asn Leu Ser Pro
420 425 430

Ser Ala Lys Arg Lys Glu Ser Lys Ser His Lys His Pro Lys Thr Val
435 440 445

Met Val Glu Asn Met Arg Ala Tyr Asp Lys Gly Asn Arg Thr Arg Ile
450 455 460

Leu Leu Gly Ser Arg Pro Pro Asn Cys Thr Asn Lys Cys His Gly Cys
465 470 475 480

Ser Pro Cys Lys Ala Lys Leu Val Ile Val His Arg Ile Met Pro Gln
485 490 495

Glu Tyr Tyr Pro Gln Arg Trp Ile Cys Ser Cys His Gly Lys Ile Tyr
500 505 510

His Pro

Sequence No. 3

Length of the sequence: 1380

Type of the sequence: nucleic acid

Topology: double stranded

Kind of the sequence: cDNA

Sequence

```
GCTATCAACA TCTTCAATGT GGAAAAATAT GGCGCAGTAG GCGATGGAAA GCATGATTGC   60
ACTGAGGCAT TTTCAACAGC ATGGCAAGCT GCATGCAAAA ACCCATCAGC AATGTTGCTT  120
GTGCCAGGCA GCAAGAAATT TGTTGTAAAC AATTTGTTCT TCAATGGGCC ATGTCAACCT  180
CACTTTACTT TTAAGGTAGA TGGGATAATA GCTGCGTACC AAAATCCAGC GAGCTGGAAG  240
AATAATAGAA TATGGTTGCA GTTTGCTAAA CTTACAGGTT TTACTCTAAT GGGTAAAGGT  300
GTAATTGATG GGCAAGGAAA ACAATGGTGG GCTGGCCAAT GTAAATGGGT CAATGGACGA  360
GAAATTTGCA ACGATCGTGA TAGACCAACA GCCATTAAAT TCGATTTTTC CACGGGTCTG  420
ATAATCCAAG GACTGAAACT AATGAACAGT CCCGAATTTC ATTTAGTTTT TGGGAATTGT  480
GAGGGAGTAA AAATCATCGG CATTAGTATT ACGGCACCGA GAGACAGTCC TAACACTGAT  540
GGAATTGATA TCTTTGCATC TAAAAACTTT CACTTACAAA AGAACACGAT AGGAACAGGG  600
GATGACTGCG TCGCTATAGG CACAGGGTCT TCTAATATTG TGATTGAGGA TCTGATTTGC  660
GGTCCAGGCC ATGGAATAAG TATAGGAAGT CTTGGGAGGG AAAACTCTAG AGCAGAGGTT  720
TCATACGTGC ACGTAAATGG GGCTAAATTC ATAGACACAC AAAATGGATT AAGAATCAAA  780
ACATGGCAGG GTGGTTCAGG CATGGCAAGC CATATAATTT ATGAGAATGT TGAAATGATA  840
AATTCGGAGA ACCCCATATT AATAAATCAA TTCTACTGCA CTTCGGCTTC TGCTTGCCAA  900
AACCAGAGGT CTGCGGTTCA AATCCAAGAT GTGACATACA AGAACATACG TGGGACATCA  960
GCAACAGCAG CAGCAATTCA ACTTAAGTGT AGTGACAGTA TGCCCTGCAA AGATATAAAG 1020
CTAAGTGATA TATCTTTGAA GCTTACCTCA GGGAAAATTG CTTCCTGCCT TAATGATAAT 1080
GCAAATGGAT ATTTCAGTGG ACACGTCATC CCTGCATGCA AGAATTTAAG TCCAAGTGCT 1140
AAGCGAAAAG AATCTAAATC CCATAAACAC CCAAAAACTG TAATGGTTGA AAATATGCGA 1200
GCATATGACA AGGGTAACAG AACACGCATA TTGTTGGGGT CGAGGCCTCC GAATTGTACA 1260
AACAAATGTC ATGGTTGCAG TCCATGTAAG GCCAAGTTAG TTATTGTTCA TCGTATTATG 1320
CCGCAGGAGT ATTATCCTCA GAGGTGGATA TGCAGCTGTC ATGGCAAAAT CTACCATCCA 1380
```

Sequence No. 4

Length of the sequence: 1542

Type of the sequence: nucleic acid

Topology: double stranded

Kind of the sequence: cDNA

Sequence

```
ATGGCCATGA AATTAATTGC TCCAATGGCC TTTCTGGCCA TGCAATTGAT TATAATGGCG   60
GCAGCAGAAG ATCAATCTGC CCAAATTATG TTGGACAGTG TTGTCGAAAA ATATCTTAGA  120
TCGAATCGGA GTTTAAGAAA AGTTGAGCAT TCTCGTCATG ATGCTATCAA CATCTTCAAT  180
GTGGAAAAAT ATGGCGCAGT AGGCGATGGA AAGCATGATT GCACTGAGGC ATTTTCAACA  240
GCATGGCAAG CTGCATGCAA AAACCCATCA GCAATGTTGC TTGTGCCAGG CAGCAAGAAA  300
TTTGTTGTAA ACAATTTGTT CTTCAATGGG CCATGTCAAC CTCACTTTAC TTTTAAGGTA  360
GATGGGATAA TAGCTGCGTA CCAAAATCCA GCGAGCTGGA AGAATAATAG AATATGGTTG  420
CAGTTTGCTA AACTTACAGG TTTTACTCTA ATGGGTAAAG GTGTAATTGA TGGGCAAGGA  480
AAACAATGGT GGGCTGGCCA ATGTAAATGG GTCAATGGAC GAGAAATTTG CAACGATCGT  540
GATAGACCAA CAGCCATTAA ATTCGATTTT TCCACGGGTC TGATAATCCA AGGACTGAAA  600
CTAATGAACA GTCCCGAATT TCATTTAGTT TTTGGGAATT GTGAGGGAGT AAAAATCATC  660
GGCATTAGTA TTACGGCACC GAGAGACAGT CCTAACACTG ATGGAATTGA TATCTTTGCA  720
TCTAAAAACT TTCACTTACA AAAGAACACG ATAGGAACAG GGGATGACTG CGTCGCTATA  780
GGCACAGGGT CTTCTAATAT TGTGATTGAG GATCTGATTT GCGGTCCAGG CCATGGAATA  840
AGTATAGGAA GTCTTGGGAG GGAAAACTCT AGAGCAGAGG TTTCATACGT GCACGTAAAT  900
GGGGCTAAAT TCATAGACAC ACAAAAATGGA TTAAGAATCA AAACATGGCA GGGTGGTTCA  960
GGCATGGCAA GCCATATAAT TTATGAGAAT GTTGAAATGA TAAATTCGGA GAACCCCATA 1020
TTAATAAATC AATTCTACTG CACTTCGGCT TCTGCTTGCC AAAACCAGAG GTCTGCGGTT 1080
CAAATCCAAG ATGTGACATA CAAGAACATA CGTGGGACAT CAGCAACAGC AGCAGCAATT 1140
CAACTTAAGT GTAGTGACAG TATGCCCTGC AAAGATATAA AGCTAAGTGA TATATCTTTG 1200
AAGCTTACCT CAGGGAAAAT TGCTTCCTGC CTTAATGATA ATGCAAATGG ATATTTCAGT 1260
GGACACGTCA TCCCTGCATG CAAGAATTTA AGTCCAAGTG CTAAGCGAAA AGAATCTAAA 1320
TCCCATAAAC ACCCAAAAAC TGTAATGGTT GAAAATATGC GAGCATATGA CAAGGGTAAC 1380
```

AGAACACGCA TATTGTTGGG GTCGAGGCCT CCGAATTGTA CAAACAAATG TCATGGTTGC 1440
AGTCCATGTA AGGCCAAGTT AGTTATTGTT CATCGTATTA TGCCGCAGGA GTATTATCCT 1500
CAGAGGTGGA TATGCAGCTG TCATGGCAAA ATCTACCATC CA 1542

Sequence No. 5

Length of the sequence: 1733

Type of the sequence: nucleic acid

Topology: linear

Kind of the sequence: cDNA

Sequence

AGTTGAGTTC GAGACAAGTA TAGAAAGAAT TTTCTTTTAT TAAAATGGCC ATGAAATTAA 60
TTGCTCCAAT GGCCTTTCTG GCCATGCAAT TGATTATAAT GGCGGCAGCA GAAGATCAAT 120
CTGCCCAAAT TATGTTGGAC AGTGTTGTCG AAAAATATCT TAGATCGAAT CGGAGTTTAA 180
GAAAAGTTGA GCATTCTCGT CATGATGCTA TCAACATCTT CAATGTGGAA AAATATGGCG 240
CAGTAGGCGA TGGAAAGCAT GATTGCACTG AGGCATTTTC AACAGCATGG CAAGCTGCAT 300
GCAAAAACCC ATCAGCAATG TTGCTTGTGC CAGGCAGCAA GAAATTTGTT GTAAACAATT 360
TGTTCTTCAA TGGGCCATGT CAACCTCACT TTACTTTTAA GGTAGATGGG ATAATAGCTG 420
CGTACCAAAA TCCAGCGAGC TGGAAGAATA ATAGAATATG GTTGCAGTTT GCTAAACTTA 480
CAGGTTTTAC TCTAATGGGT AAAGGTGTAA TTGATGGGCA AGGAAAACAA TGGTGGGCTG 540
GCCAATGTAA ATGGGTCAAT GGACGAGAAA TTTGCAACGA TCGTGATAGA CCAACAGCCA 600
TTAAATTCGA TTTTTCCACG GGTCTGATAA TCCAAGGACT GAAACTAATG AACAGTCCCG 660
AATTTCATTT AGTTTTTGGG AATTGTGAGG GAGTAAAAAT CATCGGCATT AGTATTACGG 720
CACCGAGAGA CAGTCCTAAC ACTGATGGAA TTGATATCTT TGCATCTAAA AACTTTCACT 780
TACAAAAGAA CACGATAGGA ACAGGGGATG ACTGCGTCGC TATAGGCACA GGGTCTTCTA 840
ATATTGTGAT TGAGGATCTG ATTTGCGGTC CAGGCCATGG AATAAGTATA GGAAGTCTTG 900
GGAGGGAAAA CTCTAGAGCA GAGGTTTCAT ACGTGCACGT AAATGGGGCT AAATTCATAG 960
ACACACAAAA TGGATTAAGA ATCAAAACAT GGCAGGGTGG TTCAGGCATG GCAAGCCATA 1020
TAATTTATGA GAATGTTGAA ATGATAAATT CGGAGAACCC CATATTAATA AATCAATTCT 1080

```
ACTGCACTTC GGCTTCTGCT TGCCAAAACC AGAGGTCTGC GGTTCAAATC CAAGATGTGA 1140
CATACAAGAA CATACGTGGG ACATCAGCAA CAGCAGCAGC AATTCAACTT AAGTGTAGTG 1200
ACAGTATGCC CTGCAAAGAT ATAAAGCTAA GTGATATATC TTTGAAGCTT ACCTCAGGGA 1260
AAATTGCTTC CTGCCTTAAT GATAATGCAA ATGGATATTT CAGTGGACAC GTCATCCCTG 1320
CATGCAAGAA TTTAAGTCCA AGTGCTAAGC GAAAAGAATC TAAATCCCAT AAACACCCAA 1380
AAACTGTAAT GGTTGAAAAT ATGCGAGCAT ATGACAAGGG TAACAGAACA CGCATATTGT 1440
TGGGGTCGAG GCCTCCGAAT TGTACAAACA AATGTCATGG TTGCAGTCCA TGTAAGGCCA 1500
AGTTAGTTAT TGTTCATCGT ATTATGCCGC AGGAGTATTA TCCTCAGAGG TGGATATGCA 1560
GCTGTCATGG CAAAATCTAC CATCCATAAT GAGATACATT GAAACTGTAT GTGCTAGTGA 1620
ATATTCTTGT GGTACAATAT TAGAACTGAT ATTGAAAATA AATCATCAAT GTTTCTAAGG 1680
CATTTATAAT AGATTATATT AATGGTTCAA AAAAAAAAAA AAAAAAAAAA AAA        1733
```

## Claims

1. An anti-allergic agent containing, as an active ingredient, a peptide comprising a monovalent B cell epitope and having the action of inhibiting the formation of cross-linking caused by binding of an allergen and an IgE antibody, the epitope being found on the allergen molecule and specifically bound to the IgE antibody.

2. The anti-allergic agent according to Claim 1, wherein the peptide is an amino acid sequence selected from the group consisting of the following (1) through (17) sequences or a partial amino acid sequence containing 3 or more residues of the (1) to (17) amino acid sequences:

(1) Glu-Phe-Arg-Ala-Asp-His-Pro-Phe-Leu-Phe,
(2) Lys-Ala-Ser-Ile-Asp-Gly-Leu-Glu-Val,
(3) Phe-Gln-Leu-Glu-Ala-Val-Phe-Glu-Ala,
(4) Ala-Asn-Gln-Asn-Thr-Lys-Thr-Ala-Lys,
(5) Lys-Ile-Glu-Ile-Lys-Ala-Ser-Ile-Asp,
(6) Ser-Ile-Asp-Gly-Leu-Glu-Val-Asp-Val,
(7) Lys-Cys-Pro-Leu-Val-Lys-Gly-Gln-Gln,
(8) Asn-Val-Pro-Lys-Ile-Ala-Pro-Lys-Ser,
(9) Pro-Lys-Ser-Glu-Asn-Val-Val-Val-Thr,
(10) Ser-Glu-Asn-Val-Val-Val-Thr-Val-Lys,
(11) Val-Val-Thr-Val-Lys-Val-Met-Gly-Asp,
(12) Ile-Ala-Thr-His-Ala-Lys-Ile-Arg-Asp,
(13) Gln-X-Lys-Trp-Val-Asn-Gly-Arg-Glu-Ile-X,
(14) Ser-Ala-Ser-Ala-X-Gln-Asn-Gln-Arg,
(15) X-Thr-Ser-Ala-Ser-Ala-X-Gln-Asn,
(16) Asn-Leu-Phe-Phe-Asn-Gly-Pro-X-Gln,
(17) Pro-Asn-X-Thr-Asn-Lys-X-His-Gly,

wherein X represents Cys or an amino acid capable of being bound to an antibody.

3. A pharmaceutical composition for the prevention and treatment of allergic diseases containing an effective amount of a peptide and a pharmaceutically acceptable carrier, the peptide comprising a monovalent B cell epitope and having the action of inhibiting the formation of cross-linking caused by binding of an allergen and an IgE antibody, the epitope being found in the allergen molecule and specifically bound to the IgE antibody.

4. The composition according to Claim 3, wherein the peptide is an amino acid sequence selected from the group consisting of the following (1) through (17) sequences or a partial amino acid sequence containing 3 or more residues of the (1) to (17) amino acid sequences:

(1) Glu-Phe-Arg-Ala-Asp-His-Pro-Phe-Leu-Phe,
(2) Lys-Ala-Ser-Ile-Asp-Gly-Leu-Glu-Val,
(3) Phe-Gln-Leu-Glu-Ala-Val-Phe-Glu-Ala,
(4) Ala-Asn-Gln-Asn-Thr-Lys-Thr-Ala-Lys,
(5) Lys-Ile-Glu-Ile-Lys-Ala-Ser-Ile-Asp,
(6) Ser-Ile-Asp-Gly-Leu-Glu-Val-Asp-Val,
(7) Lys-Cys-Pro-Leu-Val-Lys-Gly-Gln-Gln,
(8) Asn-Val-Pro-Lys-Ile-Ala-Pro-Lys-Ser,
(9) Pro-Lys-Ser-Glu-Asn-Val-Val-Val-Thr,
(10) Ser-Glu-Asn-Val-Val-Val-Thr-Val-Lys,
(11) Val-Val-Thr-Val-Lys-Val-Met-Gly-Asp,
(12) Ile-Ala-Thr-His-Ala-Lys-Ile-Arg-Asp,
(13) Gln-X-Lys-Trp-Val-Asn-Gly-Arg-Glu-Ile-X,
(14) Ser-Ala-Ser-Ala-X-Gln-Asn-Gln-Arg,
(15) X-Thr-Ser-Ala-Ser-Ala-X-Gln-Asn,
(16) Asn-Leu-Phe-Phe-Asn-Gly-Pro-X-Gln,
(17) Pro-Asn-X-Thr-Asn-Lys-X-His-Gly,

wherein X represents Cys or an amino acid capable of being bound to an antibody.

5. A method for preventing and treating allergic diseases characterized by administering an effective amount of a peptide comprising a monovalent B cell epitope and having the action of inhibiting the formation of cross-linking caused by binding of an allergen and an IgE antibody, the epitope being found in the allergen molecule and specifically bound to the IgE antibody.

6. The method according to Claim 5, wherein the peptide is an amino acid sequence selected from the group consisting of the following (1) through (17) sequences or a partial amino acid sequence containing 3 or more residues of the (1) to (17) amino acid sequences:

(1) Glu-Phe-Arg-Ala-Asp-His-Pro-Phe-Leu-Phe,
(2) Lys-Ala-Ser-Ile-Asp-Gly-Leu-Glu-Val,
(3) Phe-Gln-Leu-Glu-Ala-Val-Phe-Glu-Ala,
(4) Ala-Asn-Gln-Asn-Thr-Lys-Thr-Ala-Lys,
(5) Lys-Ile-Glu-Ile-Lys-Ala-Ser-Ile-Asp,
(6) Ser-Ile-Asp-Gly-Leu-Glu-Val-Asp-Val,
(7) Lys-Cys-Pro-Leu-Val-Lys-Gly-Gln-Gln,
(8) Asn-Val-Pro-Lys-Ile-Ala-Pro-Lys-Ser,
(9) Pro-Lys-Ser-Glu-Asn-Val-Val-Val-Thr,
(10) Ser-Glu-Asn-Val-Val-Val-Thr-Val-Lys,
(11) Val-Val-Thr-Val-Lys-Val-Met-Gly-Asp,
(12) Ile-Ala-Thr-His-Ala-Lys-Ile-Arg-Asp,
(13) Gln-X-Lys-Trp-Val-Asn-Gly-Arg-Glu-Ile-X,
(14) Ser-Ala-Ser-Ala-X-Gln-Asn-Gln-Arg,
(15) X-Thr-Set-Ala-Ser-Ala-X-Gln-Asn,
(16) Asn-Leu-Phe-Phe-Asn-Gly-Pro-X-Gln,
(17) Pro-Asn-X-Thr-Asn-Lys-X-His-Gly,

wherein X represents Cys or an amino acid capable of being bound to an antibody.

7. Use, as a medicine, of a peptide comprising a monovalent B cell epitope and having the action of inhibiting the formation of cross-linking caused by binding of an allergen and an IgE antibody, the epitope being found in the allergen molecule and specifically bound to the IgE antibody.

8. The use according to Claim 7, wherein the peptide is an amino acid sequence selected from the group consisting of the following (1) through (17) sequences or a partial amino acid sequence containing 3 or more residues of the (1) to (17) amino acid sequences:

(1) Glu-Phe-Arg-Ala-Asp-His-Pro-Phe-Leu-Phe,
(2) Lys-Ala-Ser-Ile-Asp-Gly-Leu-Glu-Val,
(3) Phe-Gln-Leu-Glu-Ala-Val-Phe-Glu-Ala,
(4) Ala-Asn-Gln-Asn-Thr-Lys-Thr-Ala-Lys,
(5) Lys-Ile-Glu-Ile-Lys-Ala-Ser-Ile-Asp,
(6) Ser-Ile-Asp-Gly-Leu-Glu-Val-Asp-Val,
(7) Lys-Cys-Pro-Leu-Val-Lys-Gly-Gln-Gln,
(8) Asn-Val-Pro-Lys-Ile-Ala-Pro-Lys-Ser,
(9) Pro-Lys-Ser-Glu-Asn-Val-Val-Val-Thr,
(10) Ser-Glu-Asn-Val-Val-Val-Thr-Val-Lys,
(11) Val-Val-Thr-Val-Lys-Val-Met-Gly-Asp,
(12) Ile-Ala-Thr-His-Ala-Lys-Ile-Arg-Asp,
(13) Gln-X-Lys-Trp-Val-Asn-Gly-Arg-Glu-Ile-X,
(14) Ser-Ala-Ser-Ala-X-Gln-Asn-Gln-Arg,
(15) X-Thr-Ser-Ala-Ser-Ala-X-Gln-Asn,
(16) Asn-Leu-Phe-Phe-Asn-Gly-Pro-X-Gln,
(17) Pro-Asn-X-Thr-Asn-Lys-X-His-Gly,

wherein X represents Cys or an amino acid capable of being bound to an antibody.

Fig. 1

The primary structure of ovoalbumin

Gly-Ser-Ile-Gly-Ala-Ala-Ser-Met-Glu-Phe-Cys-The-Asp-Val-Phe-Lys-Glu-

Leu-Lys-Val-His-His-Ala-Asn-Glu-Asn-Ile-Phe-Tyr-Cys-Pro-Ile-Ala-Ile-Met-Ser-Ala-

Leu-Ala-Met-Val-Tyr-Leu-Gly-Ala-Lys-Asp-Ser-Thr-Arg-Thr-Gln-Ile-Asn-Lys-Val-Val-

Arg-Phe-Asp-Lys-Leu-Pro-Gly-Phe-Gly-Asp-Ser-Ile-Glu-Ala-Gln-Cys-Gly-Thr-Ser-Val-

Asn-Val-His-Ser-Ser-Leu-Arg-Asp-Ile-Leu-Asn-Gln-Ile-Thr-Lys-Pro-Asn-Asp-Val-Tyr-

Ser-Phe-Ser-Leu-Ala-Ser-Arg-Leu-Tyr-Ala-Glu-Glu-Arg-Tyr-Pro-Ile-Leu-Pro-Glu-

Tyr-Leu-Gln-Cys-Val-Lys-Glu-Leu-Tyr-Arg-Gly-Gly-Leu-Glu-Pro-Ile-Asn-Phe-Gln-

Thr-Ala-Ala-Asp-Gln-Ala-Arg-Glu-Leu-Ile-Asn-Ser-Trp-Val-Glu-Ser-Gln-Thr-Asn-Gly-Ile-

Ile-Arg-Asn-Val-Leu-Gln-Pro-Ser-Ser-Val-Asp-Ser-Gln-Thr-Ala-Met-Val-Leu-Val-Asn-

Ala-Ile-Val-Phe-Lys-Gly-Leu-Trp-Glu-Lys-Thr-Phe-Lys-Asp-Glu-Asp-Thr-Gln-Ala-Met-

Pro-Phe-Arg-Val-Thr-Glu-Gln-Glu-Ser-Lys-Pro-Val-Gln-Met-Met-Tyr-Gln-Ile-Gly-Leu-

Phe-Arg-Val-Ala-Ser-Met-Ala-Ser-Glu-Lys-Met-Lys-Ile-Leu-Glu-Leu-Pro-Phe-Ala-Ser-

Gly-Phr-Met-Ser-Met-Leu-Val-Leu-Leu-Pro-Asp-Glu-Val-Ser-Gly-Leu-Glu-Gln-Leu-Glu-

Ser-Ile-Ile-Asn-Phe-Glu-Lys-Leu-Thr-Glu-Trp-Thr-Ser-Ser-Asn-Val-Met-Glu-Glu-Arg-

Lys-Ile-Lys-Val-Tyr-Leu-Pro-Arg-Met-Lys-Met-Glu-Glu-Lys-Tyr-Asn-Leu-Thr-Ser-Val-

Leu-Met-Ala-Met-Gly-Ile-Thr-Asp-Val-Phe-Ser-Ser-Ser-Ala-Asn-Leu-Ser-Gly-

Ile-Ser-Ser-Ala-Glu-Ser-Leu-Lys-Ile-Ser-Gln-Ala-Val-His-Ala-Ala-His-Ala-

347

Glu-Ile-Asn-Glu-Ala-Gly-Arg-Glu-Val-Val-Gly-Ser-Ala-Glu-Ala-Gly-Val-Asp-

Ala-Ala-Ser-Val-Ser-Glu-Glu-Phe-Arg-Ala-Asp-His-Pro-Phe-Leu-Phe-

Cys-Ile-Lys-His-Ile-Ala-Thr-Asn-Ala-Val-Leu-Phe-Phe-Gly-Arg-Cys-

385

Val-Ser-Pro

Fig. 2

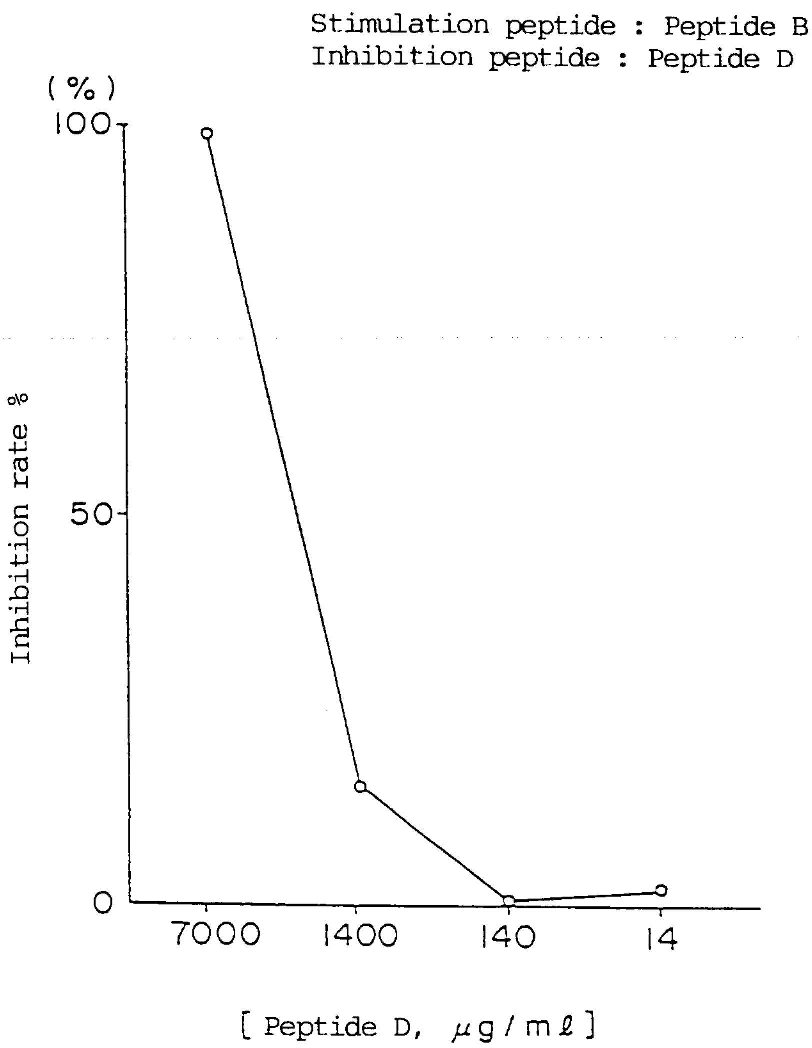
Stimulation peptide : Peptide B
Inhibition peptide : Peptide D
(%)
100
50
0
Inhibition rate %
7000
1400
140
14
[ Peptide D, μg / mℓ ]

Fig. 3

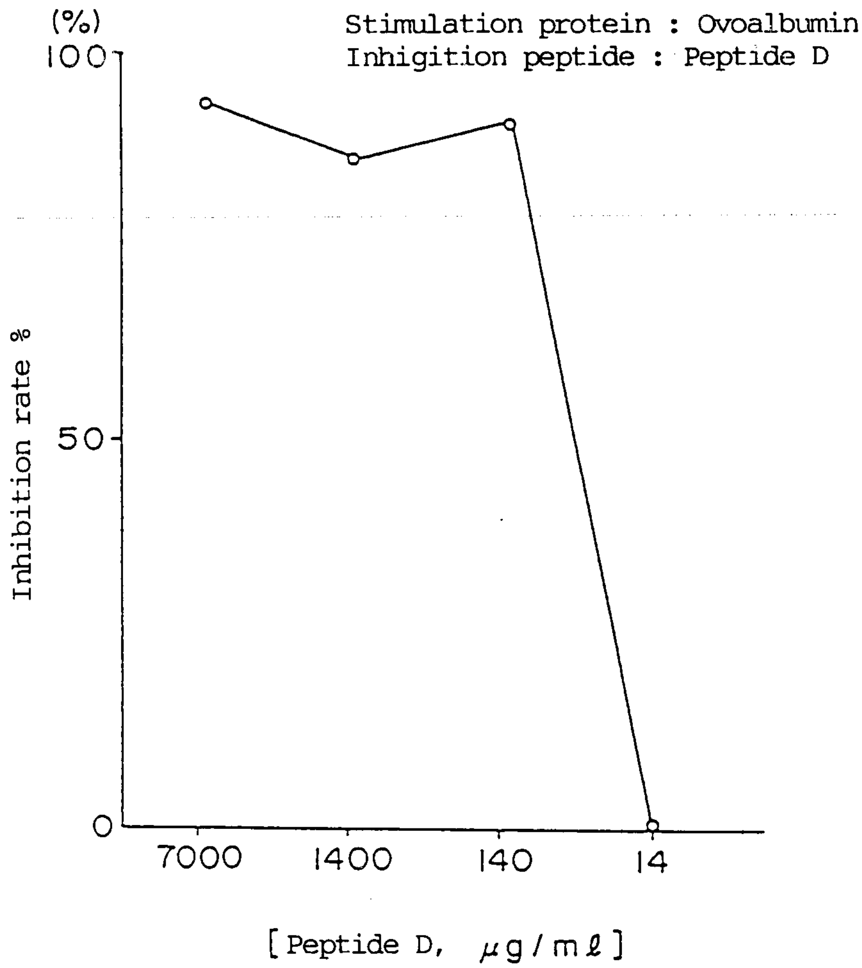
(%)
Stimulation protein : Ovoalbumin
Inhigition peptide : Peptide D
100
50
0
Inhibition rate %
7000
1400
140
14
[Peptide D, μg/mℓ]

Fig. 4

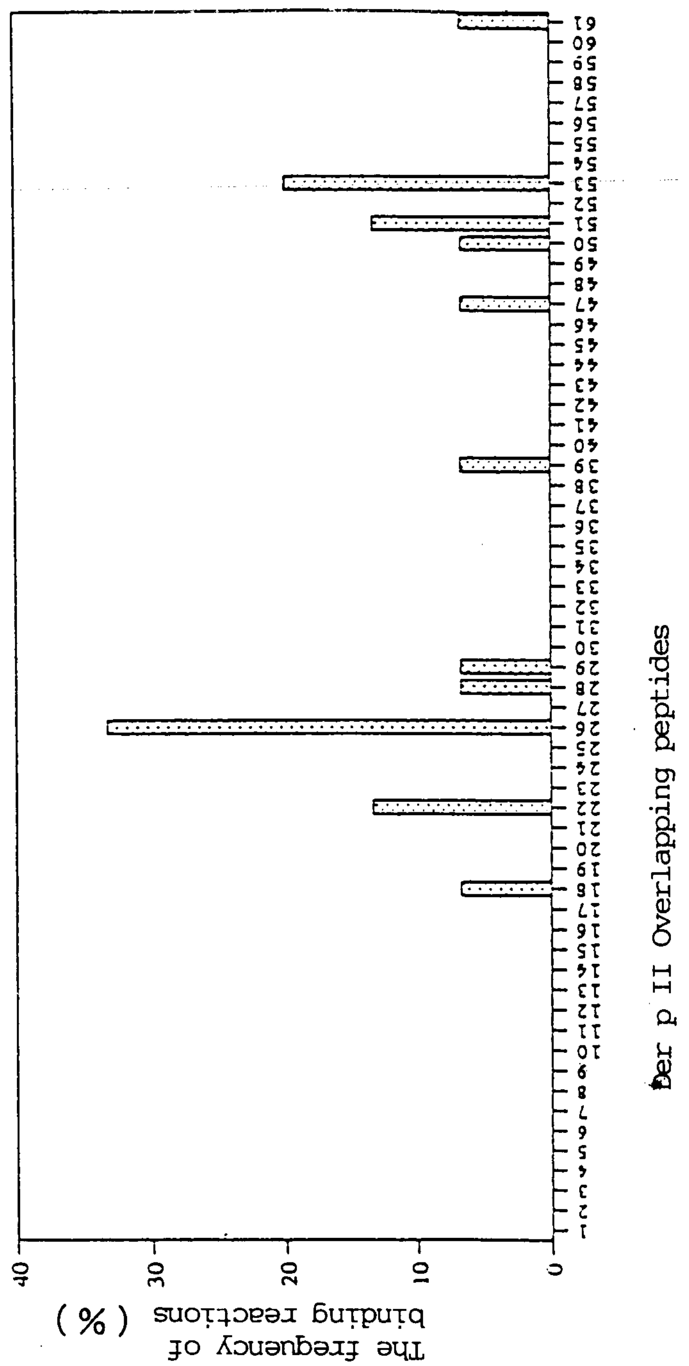

The frequency of binding reactions (%)
0
10
20
30
40
Der p II Overlapping peptides

Fig. 5

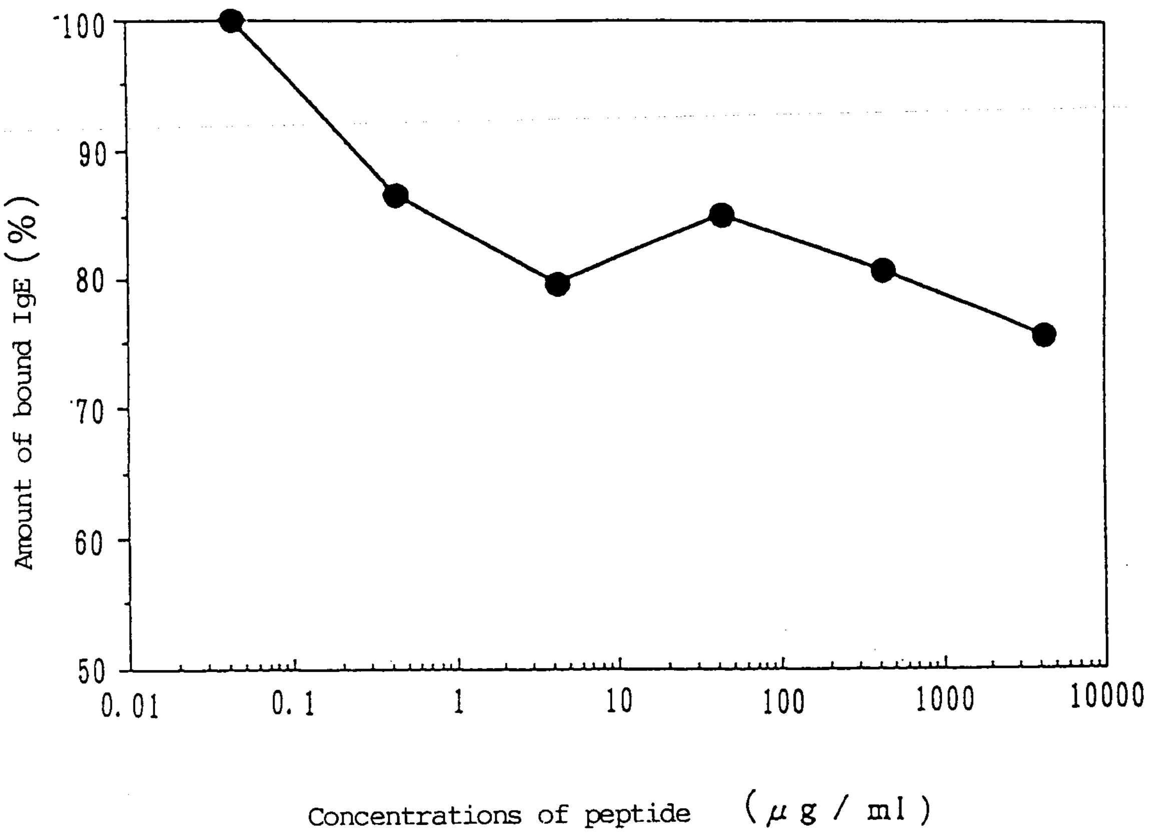
100
90
80
70
60
50
Amount of bound IgE (%)
0.01
0.1
1
10
100
1000
10000
Concentrations of peptide (μg / ml)

Fig. 6

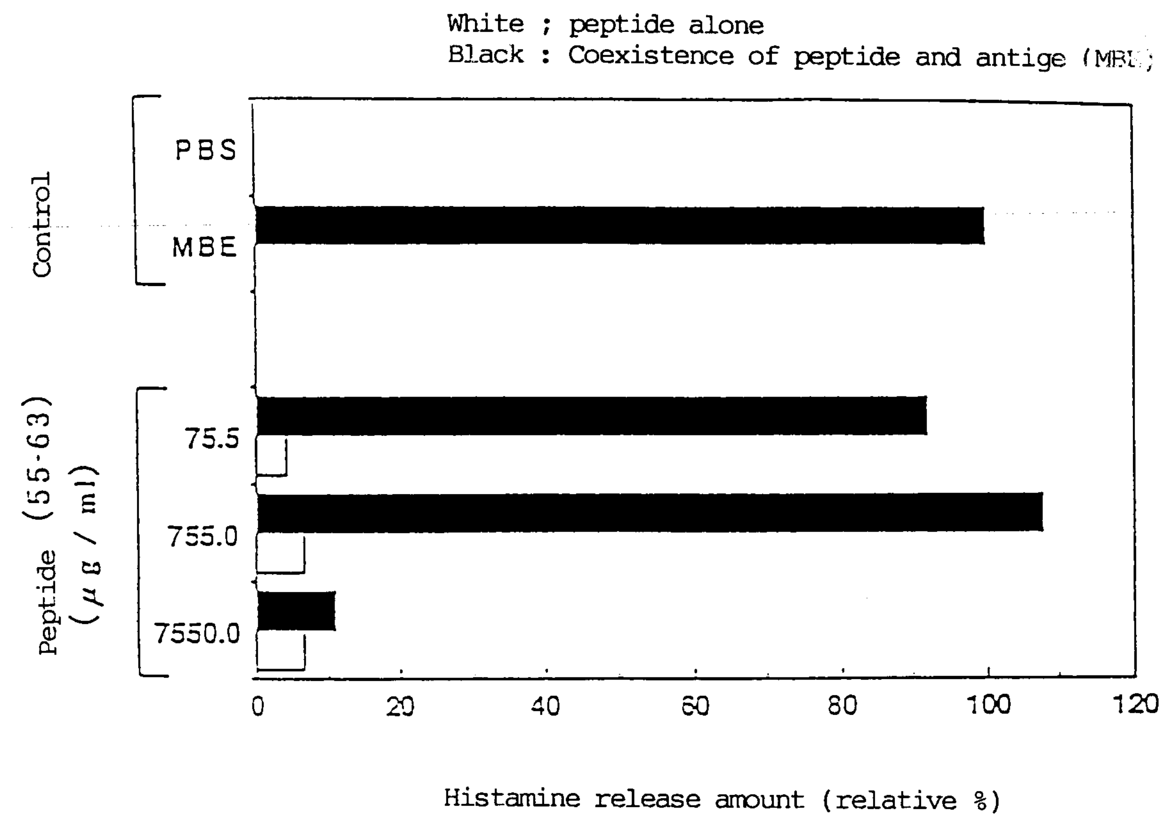
White ; peptide alone
Black : Coexistence of peptide and antige (MBE)
Control
PBS
MBE
Peptide (55-63)
(μg / ml)
75.5
755.0
7550.0
0
20
40
60
80
100
120
Histamine release amount (relative %)

Fig. 7

| | |
|---|---|
| (A) Amino acid sequence | Ala Ile Asn Ile Phe Asn Val Glu Lys Tyr |
| (B) cDNA sequence expected | 5' GCT ATT AAT ATT TTT AAT GTT GAA AAA TAT 3' |
| | 5' GCC ATC AAC ATC TTC AAC GTC GAG AAG TAC 3' |
| | 5' GCA ATA AAT ATA TTT AAT GTA GAA AAA TAT 3' |
| | 5' GCG ATT AAT ATT TTT AAT GTG GAA AAA TAT 3' |
| (C) Synthesized probe | 3' CGG TAG TTG TAG AAG TTG CAG CTT TTT ATG 5' |
| | 3' CGT TAT TTG TAT AAG TTG CAC CTT TTT ATG 5' |

Fig. 8

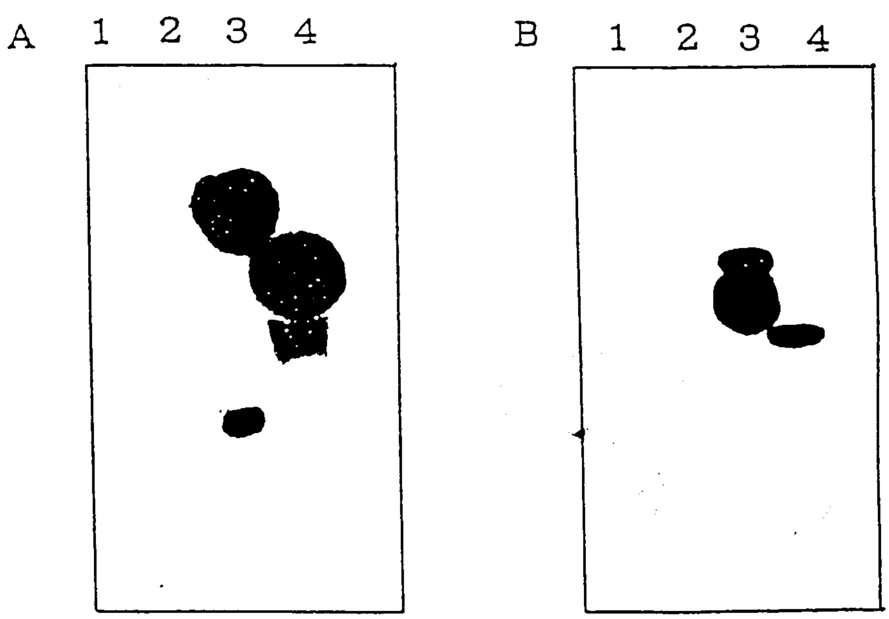


1. BL-21 holding pGEMEX-1
2. BL-21 expressing T7-CryjI
3. BL-21 expressing T7-CryjII
4. CryjI purified from cryptomeria pollens

## INTERNATIONAL SEARCH REPORT

International application No. PCT/JP94/01164

**A. CLASSIFICATION OF SUBJECT MATTER**

Int. $Cl^5$ A61K37/02, 39/35, 39/36

According to International Patent Classification (IPC) or to both national classification and IPC

**B. FIELDS SEARCHED**

Minimum documentation searched (classification system followed by classification symbols)

Int. $Cl^5$ A61K37/02, 39/35, 39/36

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, A, 5-125099 (Asahi Breweries, Ltd. and another), May 21, 1993 (21. 05. 93), (Family: none) | 1-4, 7, 8 |
| P | JP, A, 6-16695 (Asahi Breweries, Ltd. and another), January 25, 1994 (25. 01. 94), (Family: none) | 1-4, 7, 8 |
| E | JP, A, 6-256390 (Fumakilla Ltd. and another), September 13, 1994 (13. 09. 94), (Family: none) | 1-4, 7, 8 |
| A | JP, A, 5-97898 (Fumakilla Ltd. and another), April 20, 1993 (20. 04. 93), (Family: none) | 1-4, 7, 8 |
| A | JP, A, 4-288100 (Fumakilla Ltd. and another), October 13, 1992 (13. 10. 92) & EP, A, 473111 & US, A, 5314991 | 1-4, 7, 8 |
| A | JP, A, 3-291299 (Hitachi Chemical Co., Ltd.), December 20, 1991 (20. 12. 91), & EP, A, 421682 & US, A, 5118669 | 1-4, 7, 8 |

☐ Further documents are listed in the continuation of Box C. ☐ See patent family annex.

- * Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| Septemer 30, 1994 (30. 09. 94) | October 18, 1994 (18. 10. 94) |
| Name and mailing address of the ISA/ Japanese Patent Office | Authorized officer |
| Facsimile No. | Telephone No. |

Form PCT/ISA/210 (second sheet) (July 1992)